# EUROPEAN PATENT APPLICATION

(11) **EP 4 559 936 A1**
(43) Date of publication of application: **28.05.2025**
(21) Application number: 23842393.3
(22) Date of filing: 20.07.2023
(51) Int. Cl.: C07K 16/26, C12N 15/13, C12N 15/63, C12N 5/10, A61K 39/395, A61P 1/16, A61P 17/02, A61P 39/02, A61P 31/00, A61P 3/10, A61P 35/00, A61P 9/04, A61P 9/10, A61P 7/10, A61P 13/12

(54) **ANTI-ADRENOMEDULIN NON-NEUTRALIZING ANTIBODY, METHOD FOR PREPARING SAME, AND USE THEREOF**

(30) Priority: 20.07.2022 CN 202210863441
(71) Applicant: SHANGHAI JEYOU PHARMACEUTICAL CO., LTD., Shanghai 201315 (CN)
(72) Inventor: ZHENG, Yuncheng, Shanghai 201315 (CN); DENG, Sujun, Shanghai 201315 (CN); YANG, Xinxiu, Shanghai 201315 (CN); WANG, Zongda, Shanghai 201315 (CN); GU, Chunyin, Shanghai 201315 (CN)
(74) Representative: Gille Hrabal Partnerschaftsgesellschaft mbB Patentanwälte
(86) International application number: PCT/CN2023/108389
(87) International publication number: WO 2024/017331

(57) **Abstract**

The present invention belongs to the fields of bioengineering and biological treatment. Disclosed are an anti-ADM monoclonal antibody or a fragment thereof and a pharmaceutical composition, a detection reagent, or a kit comprising the monoclonal antibody or the fragment thereof. Further disclosed is use of the monoclonal antibody or the fragment thereof in the preparation of a drug.

## Description

The present application claims priority to the prior application with the patent application No. 202210863441.9 and entitled "ANTI-ADRENOMEDULLIN NON-NEUTRALIZING ANTIBODY, METHOD FOR PREPARING SAME, AND USE THEREOF" filed with the China National Intellectual Property Administration on July 20, 2022, which is incorporated herein by reference in its entirety.

### TECHNICAL FIELD

The present disclosure relates to an anti-adrenomedullin non-neutralizing antibody, a method for preparing same, and use thereof, and relates to the fields of bioengineering and biotherapy.

### BACKGROUND

Adrenomedullin (ADM) is a 6 kDa polypeptide consisting of 52 amino acids, which was first discovered in pheochromocytomas in 1993, and was later discovered to be widely secreted and expressed in vascular epidermal cells and vascular smooth muscle cells. The gene for adrenomedullin is located on chromosome 11. After translation, preprohormone is gradually digested to form calcitonin gene-related peptide (CLR). The heterodimer composed of CLR and RAMP2 or RAMP3 is a receptor for adrenomedullin which has a half-life of only 22 min based on its receptor-mediated endocytosis and protease action.

Adrenomedullin has the effects of maintaining endothelial cell integrity and enhancing the barrier. This is achieved by the following two aspects: (1) strengthening the activity of GTPase Rac1 to increase the generation of cortactin and stress fibers; and (2) reducing myosin light-chain kinase-induced actomyosin contraction by inhibiting the RhoA/ROCK pathway. Adrenomedullin also has the effects of vasodilation and lowering blood pressure. This is achieved by the following two aspects: (1) releasing NO through the PI3K/Akt pathway to activate cyclic guanosine monophosphate (cGMP)/activate protein kinase K (PKG); and (2) causing an increase in the concentration of cyclic adenosine monophosphate (cAMP)/activated protein kinase A (PKA) by binding to vascular smooth muscle, and causing relaxation of smooth muscle cells through phosphorylation.

In healthy people, the concentration of adrenomedullin is extremely low, about 10 pg/mL, and adrenomedullin can flexibly move in and out of blood vessels, flexibly adjust vasodilation, and maintain the barrier function of endothelial cells. In patients with septic shock, the concentration of adrenomedullin is 5-6 times that under normal conditions, which is directly related to the severity of the disease and prognosis. In patients with septic shock, inflammation-based effects weaken the barrier function of blood vessels, and vasodilation leads to a further reduction in blood pressure.

Adrenomedullin non-neutralizing antibodies restrict adrenomedullin in blood vessels, correct the barrier function in blood vessels, weaken the extracellular vasodilatory effect, and increase the half-life of adrenomedullin in plasma, so as to achieve the treatment and weakening of shock symptoms of sepsis. Therefore, there is an urgent need to develop a non-neutralizing antibody with a relatively high affinity for adrenomedullin.

### SUMMARY

In order to solve the technical problems described above, the present disclosure provides an anti-adrenomedullin (anti-ADM) monoclonal antibody or a fragment thereof, wherein the monoclonal antibody or the fragment specifically binds to a sequence of amino acids from position 1 to position 21 at the N-terminus of human adrenomedullin (ADM), the sequence of the amino acids from position 1 to position 21 at the N-terminus of the human ADM is set forth in SEQ ID NO: 2, and the monoclonal antibody or the fragment exhibits an affinity for ADM with a KD value of less than 10⁻¹⁰ M.

Throughout the specification of the present disclosure, the "antibody" or "antibody fragment" of the present disclosure is capable of binding to ADM, and thus is directed against ADM. Therefore, it may be referred to as an "anti-ADM antibody" or "anti-ADM antibody fragment".

The "antibody" or "antibody fragment" of the present disclosure is a non-neutralizing antibody or a fragment thereof directed against adrenomedullin.

In this context, for the sake of simplicity, an antibody or an antibody fragment with "non-neutralizing anti-ADM activity", collectively referred to as a "non-neutralizing" anti-ADM antibody or an antibody fragment (which blocks, for example, less than 80% of the biological activity of ADM), is defined as:
one or more molecules that bind to ADM, which, after adding to a culture of a eukaryotic cell line, reduce the amount of cAMP produced by the cell line through the action of a synthetic human ADM peptide added in parallel, the cell line expressing a functional human recombinant ADM receptor consisting of CRLR (calcitonin receptor-like receptor) and RAMP3 (receptor activity-modifying protein 3), wherein the added synthetic human ADM is added in an amount that results in half-maximal stimulation of cAMP synthesis in the absence of the non-neutralizing antibody to be analyzed, wherein the molecule binding to ADM results in a reduction of cAMP to an extent of no more than 80%, even when the non-neutralizing molecule to be analyzed capable of binding to ADM is added in an amount greater than 10 times the amount (required to obtain the maximum reduction in cAMP that can be obtained with the non-neutralizing antibody to be analyzed).

The same definition applies to other ranges: 95%, 90%, 50%, etc.

The biological activity is defined as the action in which a substance appears as a living organism or tissue or organ or functional unit *in vivo* or *in vitro* (e.g., in an assay) after its interaction. In terms of the biological activity of ADM, this may be the action of ADM in a human recombinant adrenomedullin receptor cAMP function assay. Therefore, according to the present disclosure, the biological activity is defined by an adrenomedullin receptor cAMP function assay.

To determine the biological activity of ADM in such an assay, the following steps may be performed:
A dose-response curve is performed using ADM in the human recombinant adrenomedullin receptor cAMP function assay.

The concentration of ADM at half-maximal cAMP stimulation may be calculated.

At constant half-maximal cAMP stimulation, the ADM concentration dose-response curve (up to a final concentration of 100 µg/mL) is performed separately by an ADM stabilizing antibody or an adrenomedullin stabilizing antibody fragment.

A maximum inhibition of 50% in the ADM bioassay indicates that the anti-ADM antibody or the anti-adrenomedullin antibody fragment each blocks 50% of the biological activity of a baseline value. A maximum inhibition of 80% in the ADM bioassay indicates that the anti-ADM antibody or the anti-adrenomedullin antibody fragment each blocks 80% of the biological activity of ADM. This is in the sense of blocking no more than 80% of the biological activity of ADM. This means that about 20% residual biological activity of ADM is still present.

However, by the present specification and in the context described above, in connection with the anti-ADM antibody and the anti-ADM antibody fragment disclosed herein, the expression "blocking the biological activity of ADM" should be understood to mean that only the biological activity of ADM is reduced, preferably reducing the biological activity of ADM from 100% to 20% residual biological activity of ADM at the maximum, preferably reducing the biological activity of ADM from 100% to 50% residual biological activity of ADM, but in any case there is still the biological activity of ADM that can be determined as described above.

Herein, the anti-adrenomedullin (ADM) antibody is an antibody that can specifically bind to ADM, and the anti-adrenomedullin antibody fragment is a fragment of an ADM antibody, wherein the fragment can specifically bind to ADM. Specific binding to ADM also allows binding to other antigens. This means that the specificity does not exclude that the antibody may cross-react with polypeptides other than the polypeptide eliciting the antibody. This is also suitable for the specificity of the anti-ADM antibody or the fragment thereof of the present disclosure.

The non-neutralizing anti-ADM antibody or the non-neutralizing anti-ADM antibody fragment of the present disclosure provides a therapeutic advantage significantly over the neutralizing anti-ADM antibody or the neutralizing anti-ADM antibody fragment. The antibody of the present disclosure is a protein including one or more polypeptides substantially encoded by immunoglobulin genes that can specifically bind to an antigen. Recognized immunoglobulin genes include κ, λ, α (IgA), γ (IgG1, IgG2, IgG3, and IgG4), δ (IgD), ε (IgE), and µ (IgM) constant region genes, as well as numerous immunoglobulin variable region genes. The length of a full-length immunoglobulin light chain is generally about 25 KDa or 214 amino acids. The length of a full-length immunoglobulin heavy chain is generally about 50 KDa or 446 amino acids. The light chain is encoded by a variable region gene (about 110 amino acids in length) located at the NH₂-terminus and a κ or λ constant region gene located at the COOH-terminus. The heavy chain is also encoded by a variable region gene (about 116 amino acids in length) and one of the other constant region genes.

The basic structural unit of an antibody is generally a tetramer composed of two pairs of identical immunoglobulin chains, each pair having one light chain and one heavy chain. In each pair, the light and heavy chain variable regions bind to an antigen, while the constant regions mediate effects or functions. Immunoglobulins also exist in a variety of other forms, including, for example, Fv, Fab and F(ab')₂, as well as bifunctional hybrid antibodies and single-chain antibodies. An immunoglobulin light or heavy chain variable region comprises a framework region interrupted by three hypervariable regions, wherein the hypervariable regions are also referred to as complementarity determining regions (CDRs). As noted above, the CDRs are primarily responsible for binding to epitopes of an antigen. An immune complex is an antibody, such as a monoclonal antibody, a chimeric antibody, a humanized antibody or a human antibody, or a functional antibody fragment, that specifically binds to an antigen.

In the art, the CDRs of an antibody can be defined by a variety of methods, such as the Kabat definition rules based on sequence variability, the Chothia definition rules based on the position of structural loop regions, and the concepts based on IMGT-ONTOLOGY. In the present disclosure, amino acid sequences of VL and VH of the anti-ADM antibody are encoded according to the Chothia numbering scheme, and the light chain CDR1-3 (LCDR 1-3) and heavy chain CDR1-3 (HCDR 1-3) of the anti-ADM antibody are defined according to the Chothia.

The chimeric antibody is an antibody whose light and heavy chain genes are constructed by genetic engineering from immunoglobulin variable and constant region genes belonging to different species. For example, a variable segment from a mouse monoclonal antibody gene may be linked to a human constant segment such as κ and γ1 or γ3. In one example, a therapeutic chimeric antibody is thus a hybrid protein composed of a variable domain or an antigen-binding domain from a mouse antibody and a constant or effector domain from a human antibody, although the variable region may be generated using other mammalian species or by molecular techniques.

"Humanized" immunoglobulin is an immunoglobulin comprising a human framework region and one or more CDRs of a non-human (such as mouse, rat, or synthetic) immunoglobulin. The non-human immunoglobulin providing the CDRs is referred to as a "donor" and the human immunoglobulin providing the framework is referred to as an "acceptor".

In one embodiment, all CDRs in the humanized immunoglobulin are derived from a donor immunoglobulin. The constant regions need not be present, but if present, they must be substantially identical, i.e., at least about 85-90%, such as about 95% identical or more identical, to human immunoglobulin constant regions. Therefore, all parts of the humanized immunoglobulin, possibly except the CDRs, are substantially identical to the corresponding parts of native human immunoglobulin sequences. "Humanized antibody" is an antibody comprising a humanized light chain and a humanized heavy chain immunoglobulin. The humanized antibody can bind to the same antigen as the donor antibody providing the CDRs. The acceptor framework of the humanized immunoglobulin or antibody may have a limited number of substitutions of amino acids from the donor framework. Humanized or other monoclonal antibodies may have other conservative amino acid substitutions that have substantially no effect on antigen binding or other immunoglobulin functions. The humanized immunoglobulin may be constructed by means of genetic engineering.

The human antibody is an antibody in which the light and heavy chain genes are derived from humans. The human antibody may be produced by immortalizing human B cells secreting an antibody of interest. The immortalization may be achieved, for example, by EBV infection or by fusing human B cells with myeloma or hybridoma cells to produce trioma cells. The human antibody may also be produced by phage display methods, or selected from human combinatorial monoclonal antibody libraries. The human antibody may also be prepared using transgenic animals carrying human immunoglobulin genes.

Therefore, the anti-ADM antibody may have a form known in the art. Examples are human antibodies, monoclonal antibodies, humanized antibodies, chimeric antibodies, and CDR-grafted antibodies.

In a preferred embodiment, the antibody of the present disclosure is a recombinantly produced antibody, such as an IgG, or an antibody fragment containing at least an F-variable domain of a heavy chain and/or light chain, such as a chemically coupled antibody (fragment antigen binding).

Therefore, in a preferred embodiment of the present disclosure, the anti-adrenomedullin monoclonal antibody fragment of the present disclosure includes a Fab, a Fab', an F(ab)₂, an Fv fragment, an F(ab')₂, a scFv, a di-scFv, a VHH, and/or a dAb.

In one preferred embodiment of the present disclosure, the antibody of the present disclosure may be produced as follows:
Mice are immunized with synthetic 16 amino acid polypeptides at the N-terminus of human ADM, 21 amino acid polypeptides at the N-terminus of human ADM or 19 amino acid polypeptides at the N-terminus of mouse ADM (YY-19 for short, SEQ ID NO: 3) and a conjugate as immunogens, respectively. One week after the last immunization, blood is collected, and the titer of serum anti-YY-21 is determined by ELISA. After mice with high serum titers are subjected to booster-immunization with immunogens, spleen cells are taken for fusion. After the fusion of spleen cells and screening for hybridoma clones, a hybridoma clone is obtained. Through the affinity assay, the hybridoma clone exhibits an affinity for ADM with a KD value of less than 10⁻¹⁰ M.

In the present disclosure, the terms "K_{D}", *"K_{D}",* and "KD" are used interchangeably and generally refer to the equilibrium dissociation constant of an antibody-antigen interaction. "KD" as used herein is the ratio of a dissociation rate constant (kdis, also referred to as "off-rate (koff)" or "kd") to an association rate constant (kon, also referred to as "on-rate (kon)" or "ka").

Through sequencing, the heavy chain variable region of the hybridoma clone comprises the following CDR sequences:
(i) GYAFTTF (set forth in SEQ ID NO: 11),
(ii) NTYSRV (set forth in SEQ ID NO: 12), and
(iii) GYGGEGGLGF (set forth in SEQ ID NO: 13); and
the light chain variable region of the hybridoma clone comprises the following CDR sequences:
(i) RSSQSIIDSDGNTYLE (set forth in SEQ ID NO: 14),
(ii) KVSNRFS (set forth in SEQ ID NO: 15), and
(iii) FQGSHFPYT (set forth in SEQ ID NO: 16).

In certain embodiments of the present disclosure, the hybridoma clone comprises a heavy chain variable region sequence set forth in SEQ ID NO: 9, and the hybridoma clone comprises a light chain variable region sequence set forth in SEQ ID NO: 10. Humanization of the anti-ADM antibody may be performed according to the following solution:
Through sequence alignment, a human antibody germline gene with the highest homology is selected as a humanization design framework. CDR grafting and back mutations are performed on the heavy chain variable region of the hybridoma clone to obtain a humanized heavy chain variable region sequence. CDR grafting and back mutations are performed on the light chain variable region of the hybridoma clone to obtain a humanized light chain variable region sequence.

Thus, in certain embodiments of the present disclosure, the heavy chain variable region of the humanized antibody comprises the following CDR sequences:
(i) GYAFTTF (set forth in SEQ ID NO: 11),
(ii) NTYSRV (set forth in SEQ ID NO: 12), and
(iii) GYGGEGGLGF (set forth in SEQ ID NO: 13); and
the light chain variable region of the humanized antibody comprises the following CDR sequences:
(i) RSSQSIIDSDGNTYLE (set forth in SEQ ID NO: 14),
(ii) KVSNRFS (set forth in SEQ ID NO: 15), and
(iii) FQGSHFPYT (set forth in SEQ ID NO: 16).

In certain embodiments of the present disclosure, the humanized antibody comprises a heavy chain variable region sequence set forth in SEQ ID NO: 17, and the humanized antibody comprises a light chain variable region sequence set forth in any one of SEQ ID NOs: 18 and 19.

In certain embodiments of the present disclosure, the humanized antibody comprises a heavy chain variable region sequence set forth in SEQ ID NO: 17, and the humanized antibody comprises a light chain variable region sequence set forth in SEQ ID NO: 18. In certain embodiments of the present disclosure, the humanized antibody comprises a heavy chain variable region sequence set forth in SEQ ID NO: 17, and the humanized antibody comprises a light chain variable region sequence set forth in SEQ ID NO: 19. In certain embodiments of the present disclosure, the monoclonal antibody further comprises a light chain constant region of the antibody, and the light chain constant region is a human Kappa chain constant region. In the present disclosure, the human Kappa chain constant region comprises an amino acid sequence set forth in SEQ ID NO: 20.

In certain embodiments of the present disclosure, the monoclonal antibody further comprises a heavy chain constant region of the antibody, and the heavy chain constant region is a human IgG1 constant region. In the present disclosure, the human IgG1 constant region comprises an amino acid sequence set forth in SEQ ID NO: 21.

The light chain variable region sequence and the human Kappa chain constant region of the monoclonal antibody of the present disclosure are combined to form a light chain of the antibody, and the heavy chain variable region sequence and the human IgG1 constant region of the monoclonal antibody of the present disclosure are combined to form a heavy chain of the antibody.

Thus, in certain embodiments of the present disclosure, the monoclonal antibody comprises a heavy chain sequence set forth in any one of SEQ ID NOs: 23, 25, and 27, and the monoclonal antibody antibody comprises a light chain sequence set forth in any one of SEQ ID NOs: 22, 24, and 26.

In certain embodiments of the present disclosure, the monoclonal antibody comprises a heavy chain sequence set forth in SEQ ID NO: 23, and the monoclonal antibody comprises a light chain sequence set forth in SEQ ID NO: 22.

In certain embodiments of the present disclosure, the monoclonal antibody comprises a heavy chain sequence set forth in SEQ ID NO: 25, and the monoclonal antibody comprises a light chain sequence set forth in SEQ ID NO: 24.

In certain embodiments of the present disclosure, the monoclonal antibody comprises a heavy chain sequence set forth in SEQ ID NO: 27, and the monoclonal antibody comprises a light chain sequence set forth in SEQ ID NO: 26.

After codon optimization, gene synthesis is performed, and the synthesized gene fragment is cloned into an expression vector. After amplification of expression plasmids and plasmid extraction, dual plasmids are co-transfected into Expi293F or CHO-K1 cells, and the antibody is transiently transfected into cells, expressed, and purified by a Protein A affinity chromatography column after the expression. Through the affinity assay, the humanized anti-ADM antibody exhibits an affinity for ADM with a KD value of less than 10⁻¹⁰ M.

The present disclosure may also screen the anti-ADM monoclonal antibody by panning of a fully human single-chain phage antibody library.

Thus, in a preferred embodiment of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SAYNGN (set forth in SEQ ID NO: 44), and
   (iii) EGRSGGSFDI (set forth in SEQ ID NO: 45); and
(b) the light chain variable region of the monoclonal antibody comprises the following CDR sequences:
   (i) RASQGISSYLA (set forth in SEQ ID NO: 46),
   (ii) DASNLET (set forth in SEQ ID NO: 47), and
   (iii) QQYDNLPLT (set forth in SEQ ID NO: 48).

In the present application, "monoclonal antibody" generally refers to an antibody obtained from a population of substantially homogeneous antibodies, i.e., the individual antibodies constituting the population are identical except for possible naturally occurring mutations and/or post-translational modifications (e.g., isomerization, amidation) that may be present in minimal amounts. For example, in certain embodiments of the present disclosure, the anti-adrenomedullin monoclonal antibody contains a post-translational modification (PTM) site of NG.

In certain embodiments of the present disclosure, the post-translational modification (PTM) site of NG contained in the anti-adrenomedullin monoclonal antibody is mutated into QG by site-directed mutagenesis to remove deamidation and isomerization effects.

Thus, in certain embodiments of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SAYQGN (set forth in SEQ ID NO: 49), and
   (iii) EGRSGGSFDI (set forth in SEQ ID NO: 45); and
(b) the light chain variable region of the monoclonal antibody comprises the following CDR sequences:
   (i) RASQGISSYLA (set forth in SEQ ID NO: 46),
   (ii) DASNLET (set forth in SEQ ID NO: 47), and
   (iii) QQYDNLPLT (set forth in SEQ ID NO: 48).

In certain embodiments of the present disclosure, the monoclonal antibody comprises a heavy chain variable region sequence set forth in any one of SEQ ID NOs: 28 and 29, and the monoclonal antibody comprises a light chain variable region sequence set forth in SEQ ID NO: 35.

In certain embodiments of the present disclosure, the humanized antibody comprises a heavy chain variable region sequence set forth in SEQ ID NO: 28, and the humanized antibody comprises a light chain variable region sequence set forth in SEQ ID NO: 35. In certain embodiments of the present disclosure, the humanized antibody comprises a heavy chain variable region sequence set forth in SEQ ID NO: 29, and the humanized antibody comprises a light chain variable region sequence set forth in SEQ ID NO: 35. In order to improve the affinity of fully human antibodies for human ADM, the present disclosure also designs and constructs an affinity maturation library of fully human antibodies. By screening the affinity maturation library of fully human antibodies and identifying monoclones, the present disclosure obtains different fully human anti-ADM antibodies, all of which exhibit an affinity for ADM with a KD value of less than 10⁻¹⁰ M.

Therefore, in a preferred embodiment of the present disclosure, in the anti-adrenomedullin monoclonal antibody or the fragment thereof:
(a) the heavy chain variable region of the monoclonal antibody comprises the following CDR sequences:
   (i) GYTFTX₁Y, wherein X₁ is selected from S, Q, and H,
   (ii) SX₂YX₃GX₄, wherein X₂ is selected from A and P, X₃ is selected from N, Q, S, and T, and X₄ is selected from N and K, and
   (iii) EGRX₅GGSFX₆I, wherein X₅ is selected from S and W, and X₆ is selected from D and N; and
(b) the light chain variable region of the monoclonal antibody comprises the following CDR sequences:
   (i) RAX₇X₈GIX₉X₁₀YLA, wherein X₇ is selected from S and A, X₈ is selected from Q and E, X₉ is selected from S and G, and X₁₀ is selected from S and E,
   (ii) DX₁₁SX₁₂X₁₃X₁₄X₁₅, wherein X₁₁ is selected from A, V, and T, X₁₂ is selected from N, I, and D, X₁₃ is selected from L and V, X₁₄ is selected from E and D, and X₁₅ is selected from T and A, and
   (iii) QQYDX₁₆LX₁₇LX₁₈, wherein X₁₆ is selected from N and D, X₁₇ is selected from P and D, and X₁₈ is selected from T and S.

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DASNLET (set forth in SEQ ID NO: 47), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTQY (set forth in SEQ ID NO: 51),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASEGISEYLA (set forth in SEQ ID NO: 52),
   (ii) DASNLET (set forth in SEQ ID NO: 47), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTQY (set forth in SEQ ID NO: 51),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RAAEGIGSYLA (set forth in SEQ ID NO: 53),
   (ii) DASNLET (set forth in SEQ ID NO: 47), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SPYSGN (set forth in SEQ ID NO: 54), and
   (iii) EGRWGGSFNI (set forth in SEQ ID NO: 50); and
(b) the light chain variable region of the monoclonal antibody comprises the following CDR sequences:
   (i) RASEGISEYLA (set forth in SEQ ID NO: 52),
   (ii) DASNLET (set forth in SEQ ID NO: 47), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTHY (set forth in SEQ ID NO: 55),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DVSILDA (set forth in SEQ ID NO: 56), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTQY (set forth in SEQ ID NO: 51),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DVSILDA (set forth in SEQ ID NO: 56), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTQY (set forth in SEQ ID NO: 51),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DASNVDT (set forth in SEQ ID NO: 57), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SPYTGK (set forth in SEQ ID NO: 58), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DVSILDA (set forth in SEQ ID NO: 56), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTSY (set forth in SEQ ID NO: 43),
   (ii) SPYTGK (set forth in SEQ ID NO: 58), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DTSDLDT (set forth in SEQ ID NO: 59), and
   (iii) QQYDNLPLT (set forth in SEQ ID NO: 48).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTHY (set forth in SEQ ID NO: 55),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DASNLET (set forth in SEQ ID NO: 47), and
   (iii) QQYDDLDLT (set forth in SEQ ID NO: 60).

In a further preferred embodiment of the present disclosure, in the anti-adrenomedullin monoclonal antibody or the fragment thereof,
(a) the heavy chain variable region of the monoclonal antibody comprises the following CDR sequences:
   (i) GYTFTQY (set forth in SEQ ID NO: 51),
   (ii) SAYQGN (set forth in SEQ ID NO: 49), and
   (iii) EGRWGGSFNI (set forth in SEQ ID NO: 50); and
(b) the light chain variable region of the monoclonal antibody comprises the following CDR sequences:
   (i) RASQGISSYLA (set forth in SEQ ID NO: 46),
   (ii) DASNLET (set forth in SEQ ID NO: 47), and
   (iii) QQYDDLPLS (set forth in SEQ ID NO: 61).

In addition, the present disclosure further obtains a heavy chain variable region sequence and a light chain variable region sequence of an antibody with an improved affinity.

Thus, in a preferred embodiment of the present disclosure, in the anti-adrenomedullin monoclonal antibody or the fragment thereof, the monoclonal antibody comprises a heavy chain variable region sequence set forth in any one of SEQ ID NOs: 30 to 34, and the monoclonal antibody comprises a light chain variable region sequence set forth in any one of SEQ ID NOs: 35 to 42.

In certain embodiments of the present disclosure, the humanized antibody comprises a heavy chain variable region sequence set forth in SEQ ID NO: 30, and the humanized antibody comprises a light chain variable region sequence set forth in SEQ ID NO: 35. In certain embodiments of the present disclosure, the humanized antibody comprises a heavy chain variable region sequence set forth in SEQ ID NO: 31, and the humanized antibody comprises a light chain variable region sequence set forth in SEQ ID NO: 36. In certain embodiments of the present disclosure, the humanized antibody comprises a heavy chain variable region sequence set forth in SEQ ID NO: 31, and the humanized antibody comprises a light chain variable region sequence set forth in SEQ ID NO: 37. In certain embodiments of the present disclosure, the humanized antibody comprises a heavy chain variable region sequence set forth in SEQ ID NO: 32, and the humanized antibody comprises a light chain variable region sequence set forth in SEQ ID NO: 36. In certain embodiments of the present disclosure, the humanized antibody comprises a heavy chain variable region sequence set forth in SEQ ID NO: 33, and the humanized antibody comprises a light chain variable region sequence set forth in SEQ ID NO: 38. In certain embodiments of the present disclosure, the humanized antibody comprises a heavy chain variable region sequence set forth in SEQ ID NO: 31, and the humanized antibody comprises a light chain variable region sequence set forth in SEQ ID NO: 38. In certain embodiments of the present disclosure, the humanized antibody comprises a heavy chain variable region sequence set forth in SEQ ID NO: 31, and the humanized antibody comprises a light chain variable region sequence set forth in SEQ ID NO: 39. In certain embodiments of the present disclosure, the humanized antibody comprises a heavy chain variable region sequence set forth in SEQ ID NO: 34, and the humanized antibody comprises a light chain variable region sequence set forth in SEQ ID NO: 38. In certain embodiments of the present disclosure, the humanized antibody comprises a heavy chain variable region sequence set forth in SEQ ID NO: 34, and the humanized antibody comprises a light chain variable region sequence set forth in SEQ ID NO: 40. In certain embodiments of the present disclosure, the humanized antibody comprises a heavy chain variable region sequence set forth in SEQ ID NO: 33, and the humanized antibody comprises a light chain variable region sequence set forth in SEQ ID NO: 41. In certain embodiments of the present disclosure, the humanized antibody comprises a heavy chain variable region sequence set forth in SEQ ID NO: 31, and the humanized antibody comprises a light chain variable region sequence set forth in SEQ ID NO: 42. In certain embodiments of the present disclosure, the monoclonal antibody further comprises a light chain constant region of the antibody, and the light chain constant region is a human Kappa chain constant region. In the present disclosure, the human Kappa chain constant region comprises an amino acid sequence set forth in SEQ ID NO: 20.

In certain embodiments of the present disclosure, the monoclonal antibody further comprises a heavy chain constant region of the antibody, and the heavy chain constant region is a human IgG1 constant region. In the present disclosure, the human IgG1 constant region comprises an amino acid sequence set forth in SEQ ID NO: 21.

The light chain variable region sequence and the human Kappa chain constant region of the monoclonal antibody are combined to form a light chain of the antibody, and the heavy chain variable region sequence and the human IgG1 constant region of the monoclonal antibody are combined to form a heavy chain of the antibody.

After codon optimization, gene synthesis is performed, the synthesized gene fragment is cloned into an expression vector pcDNA3.4, and expressed and purified in CHO cells, and an affinity-matured monoclonal antibody can be obtained. Through the affinity assay, the anti-ADM antibody exhibits an affinity for ADM with a KD value of less than 10⁻¹⁰ M.

In a further aspect, the present disclosure provides a nucleotide sequence encoding the anti-adrenomedullin monoclonal antibody or the fragment thereof of the present disclosure.

In a further aspect, the present disclosure provides an expression vector comprising the nucleotide sequence of the present disclosure, wherein preferably, the expression vector is a vector pcDNA3.4.

In a further aspect, the present disclosure provides a host cell comprising the nucleotide sequence or the expression vector of the present disclosure, wherein preferably, the host cell is a CHO cell.

In a further aspect, the present disclosure provides a pharmaceutical composition, a detection reagent, or a kit comprising the anti-adrenomedullin monoclonal antibody or the fragment thereof of the present disclosure.

In a further aspect, the present disclosure provides use of the anti-adrenomedullin monoclonal antibody or the fragment thereof of the present disclosure for manufacturing a medicament for the maintenance of endothelial cell integrity and the enhancement of a barrier function.

In a further aspect, the present disclosure provides use of the anti-adrenomedullin monoclonal antibody or the fragment thereof of the present disclosure for manufacturing a medicament for controlling vasodilation and lowering blood pressure. In a further aspect, the present disclosure provides use of the anti-adrenomedullin monoclonal antibody or the fragment thereof of the present disclosure for manufacturing a medicament for the treatment or prevention of septic shock or traumatic injury, especially an advanced stage of sepsis.

In a further aspect, the present disclosure provides use of the anti-adrenomedullin monoclonal antibody or the fragment thereof of the present disclosure for manufacturing a medicament for the reduction of death risk in a patient with a chronic or acute disease or an acute condition, wherein the chronic or acute disease or the acute condition is selected from a severe infection such as meningitis, systemic inflammatory response syndrome (SIRS), and septicemia; other diseases such as diabetes, cancers, acute and chronic vascular diseases (such as heart failure, myocardial infarction, stroke, and atherosclerosis), and edema; and shock such as septic shock, organ dysfunction such as renal dysfunction and liver dysfunction, burns, surgery, trauma, and poisoning. In a further aspect, the present disclosure provides use of the anti-adrenomedullin monoclonal antibody or the fragment thereof of the present disclosure for manufacturing a medicament for the stabilization of circulation, especially systemic circulation, in a patient, wherein the patient suffers from a chronic or acute disease or an acute condition, and the chronic or acute disease or the acute condition is selected from a severe infection such as meningitis, systemic inflammatory response syndrome (SIRS), and septicemia; other diseases such as diabetes, cancers, acute and chronic vascular diseases (such as heart failure, myocardial infarction, stroke, and atherosclerosis), and edema; and shock such as septic shock, organ dysfunction such as renal dysfunction and liver dysfunction, burns, surgery, trauma, and poisoning.

In a preferred embodiment of the present disclosure, the anti-adrenomedullin monoclonal antibody or the fragment thereof is used in combination with another medicament.

In a further preferred embodiment of the present disclosure, the medicament is selected from a vasopressor, an intravenous injection, a TNF-α-antibody, and an antibiotic.

### Technical Effects

The present disclosure screens the anti-ADM monoclonal antibody by hybridoma technology, and obtains the fully human anti-ADM monoclonal antibody by humanized expression of the antibody and panning of the fully human single-chain phage antibody library. On this basis, the present disclosure finally obtains relatively high binding activity to the N-terminus of human ADM by screening the affinity maturation library. The anti-ADM non-neutralizing antibody of the present disclosure exhibits an affinity for ADM with a KD value of less than 10⁻¹⁰ M compared with the anti-ADM non-neutralizing antibodies in the prior art.

Animal experiments have shown that treatment with the anti-ADM non-neutralizing antibody of the present disclosure can significantly improve symptoms of sepsis and improve the survival rates of animals.

### BRIEF DESCRIPTION OF THE DRAWINGS

FIG. 1 is a schematic diagram showing anti-ADM antibodies blocking hADM-induced cAMP production in CHOK1/CRLR/RAMP3 cells.
FIG. 2 is a graph showing the survival rates of mice with LPS-induced sepsis affected by anti-ADM antibodies.

### DETAILED DESCRIPTION

For better illustrating the objectives and advantages of the present method, the detailed description of the present disclosure will be further described in detail with reference to the accompanying drawings and specific examples.

### Example 1: Screening of Anti-ADM Monoclone by Hybridoma Technology

### 1.1 Immunization of mice

Mice were immunized with a conjugate of 16 amino acid polypeptides at the N-terminus of synthetic human ADM (YY-16 for short, SEQ ID NO: 1), 21 amino acid polypeptides at the N-terminus of human ADM (YY-21 for short, SEQ ID NO: 2) or 19 amino acid polypeptides at the N-terminus of mouse ADM (YY-19 for short, SEQ ID NO: 3) and KLH (Sigma, H8283) as an immunogen. At the time of primary immunization, the immunogen and Freund's complete adjuvant were emulsified in a ratio of 1:1, and the mixture was intraperitoneally injected into female Balb/c mice, SJL mice, or SD rats aged 6-8 weeks, with 100 µg/mouse. Subsequently, booster immunization was performed every 2-3 weeks, and each animal was given the immunogen and Freund's incomplete adjuvant (50 µg). After 3-4 times of immunization, blood was collected to measure the titer. One week after the last immunization, blood was collected, and the titer of serum anti-YY-21 was determined by ELISA. Mice with high serum titers were intraperitoneally injected with 50 µg of the immunogen for intensive immunization, and the spleen cells of animals were taken for fusion on the third day.

### 1.2 Fusion of spleen cells

After euthanasia, the mice were dissected. The spleen was taken and ground, and the cells were collected, suspended in 5 mL of red blood cell lysis buffer, and placed at 4 °C for 5 min. The reaction was terminated with DMEM + 10% FBS. After centrifugation, the spleen cells were resuspended in 40 mL of DMEM, and the suspension was left to stand for 2-3 min. The supernatant was then transferred to another 50 mL centrifuge tube. SP2/0 and spleen cells were mixed in a ratio of 1:2, and the mixture was centrifuged. The supernatant was sufficiently pipetted, and the cell pellet was mixed. The mixed cells were washed twice with DMEM, and resuspended in an electrofusion buffer. The suspension was added to an electrofusion tank. After the electrofusion procedure was completed, the fused cells were first left to stand for 5 min, and then added to a DMEM + 10% FBS + 1 × HAT screening medium. The cell suspension described above was added to a 96-well cell culture plate, and cultured in an incubator at 37 °C with 75% humidity and 5% CO₂ for 7-9 days.

### 1.3 Screening of hybridoma clone

YY-21 or YY-19 was diluted with PBS to 1.0 µg/mL, added to a 96-well plate (Corning, 9018), with 100 µL/well, and incubated at 4 °C overnight for coating. The ELISA plate was washed 3 times with a washing buffer (PBS + 0.05% Tween 20) on an automatic plate washer the next day. 300 µL of a blocking buffer (PBS + 0.05% Tween 20 + 1% BSA) was added to each well, and the plate was placed at room temperature for 1 h for blocking. The plate was then washed 3 times with the washing buffer on the automatic plate washer, and the hybridoma supernatant was added to each well of the ELISA plate. The plate was placed and incubated at room temperature for 1 h, and then washed 3 times according to the method described above. Goat Anti-mouse IgG Fc-HRP (Sigma, A0168) or Goat Anti-rat-IgG-HRP (Sigma, A5795) was diluted in 1:5000 with the blocking buffer, with 100 µL/well. The plate was placed and incubated at room temperature for 1 h. The plate was then washed 3 times according to the method described above. A TMB substrate solution was added with 100 µL/well and incubated at room temperature for 10 min, then 50 µL of 1.0 M hydrochloric acid was added to each well to terminate the reaction, and the plate was read by a microplate reader at OD₄₅₀ nm. Positive cells were picked for subcloning and subclone screening until a stable hybridoma cell strain that could secrete monoclonal antibodies binding to YY-21 and YY-19 was obtained. A hybridoma clone 40E12 was screened through a binding experiment. The cell strain was cryopreserved, and a serum-free medium was used for small-scale production in 50 mL. The cell strain was purified by a protein A column for subsequent identification.

### 1.4 Affinity assay of hybridoma clone 40E12

The affinity of the candidate antibody for biotin-labeled human ADM (human ADM-C-biotin for short, the sequence of human ADM is set forth in SEQ ID NO: 4) and mouse ADM (mouse ADM-C-biotin for short, the sequence of mouse ADM is set forth in SEQ ID NO: 5) was determined by adopting Octet RED96e (Fortebio). The antigens and antibody were all diluted with 1× PBST, the concentration of the antigen used was 2 µg/mL, and the working concentration of the antibody used was 100 nM.

First, the sample was added to a 96-well plate (Greiner bio-one, 655209) with a system of 200 µL/well. Software parameters were then set, the plate temperature was set to 30 °C, and the standard kinetic signal was collected at a frequency of 5.0 HZ. Next, a streptavidin sensor (Fortébio, Cat. No. 18-5020) was pre-wetted with 1× PBST for 10 min and then tested on a machine. Each cycle included the following steps: 1) immersing the sensor in a buffer for 180 s to stabilize the baseline; 2) immobilizing the antigen for 10 s, allowing the antigen to bind to the sensor, and controlling the binding amount of the antigen between 0.5 nm and 1.0 nm; 3) immersing the sensor in the buffer for 180 s; 4) binding the antigen to the antibody for the binding time of 180 s; and 5) dissociating the antigen and the antibody for 10 min.

The equilibrium dissociation constant (KD) of the antibody was calculated by measuring the association rate (Kon) and the dissociation rate (Koff) of the antigen-antibody in a 1:1 binding manner by adopting the Data Analysis 12.0 software from Fortebio. The results are shown in Table 1.

**Table 1. Equilibrium dissociation constants (KD) of candidate antibody 40E12**

| Antigen | KD (M) | Kon (1/Ms) | Koff (1/s) |
|---|---|---|---|
| Human ADM-C-biotin | 3.14E-10 | 3.47E+05 | 1.09E-04 |
| Mouse ADM-C-biotin | 2.36E-10 | 3.77E+05 | 8.90E-05 |

### 1.5 Sequencing of hybridoma clone 40E12

The hybridoma monoclonal cell strain was cultured, and 5 × 10⁶ hybridoma cells were collected by centrifugation. The total RNA was extracted by the Trizol method, the cDNA obtained after a reverse transcription reaction was subjected to G addition reaction by a terminal transferase, and then the DNA containing variable region sequences was amplified by using a VH primer (with a sequence set forth in SEQ ID NO: 6), a VK primer (with a sequence set forth in SEQ ID NO: 7), and a polyC primer (with a sequence set forth in SEQ ID NO: 8) to perform TA cloning. After sequencing, the sequences of a 40E12 heavy chain variable region (40E12VH) and a 40E12 light chain variable region (40E12VK) of the murine hybridoma clone were obtained and set forth in SEQ ID NO: 9 and SEQ ID NO: 10, respectively. The light and heavy chain variable regions were numbered according to Chothia, and the amino acid sequences of HCDR1, HCDR2, and HCDR3 of the clone 40E12 defined by adopting Chothia are set forth in SEQ ID NO: 11, SEQ ID NO: 12, and SEQ ID NO: 13, respectively, and the amino acid sequences of LCDR1, LCDR2, and LCDR3 are set forth in SEQ ID NO: 14, SEQ ID NO: 15, and SEQ ID NO: 16, respectively.

### Example 2: Humanization and Expression of Anti-ADM Antibodies

### 2.1 Humanization of anti-ADM antibodies

Through sequence alignment, a human antibody germline gene with the highest homology was selected as a humanization design framework.

For the heavy chain variable region, human antibody germline gene sequences IGHV7-4-1*02 and IGHJ6*01 were used as frameworks, and 40E12VH (also 2004hzVH0 for short) was subjected to CDR grafting and back mutations to obtain a humanized heavy chain variable region sequence 2004hzVH9 (with an amino acid sequence set forth in SEQ ID NO: 17). For the light chain variable region, human antibody germline gene sequences IGKV2-30*02 and IGKJ2*01 were used as frameworks, and 40E12VK (also 2004hzVK0 for short) was subjected to CDR grafting and back mutations to obtain humanized light chain variable region sequences 2004hzVK7 (with an amino acid sequence set forth in SEQ ID NO: 18) and 2004hzVK9 (with an amino acid sequence set forth in SEQ ID NO: 19).

### 2.2 Expression of anti-ADM antibodies

The 2004hzVK7 and 2004hzVK9 sequences were separately combined with the human Kappa chain constant region (CL, with an amino acid sequence set forth in SEQ ID NO: 20) to form antibody light chains, and the 2004hzVH9 sequence was combined with the human IgG1 constant region (CH, with an amino acid sequence set forth in SEQ ID NO: 21) to form an antibody heavy chain. 2004hzVH0 and 2004hzVK0 were combined with the human heavy chain constant region and the human light chain constant region, respectively, and then paired with each other to form a chimeric antibody 2004hz00 (with sequences set forth in SEQ ID NOs: 22 and 23); 2004hzVH9 and 2004hzVK7 were combined with the human heavy chain constant region and the human light chain constant region, respectively, and then paired with each other to form a humanized antibody 2004hz97 (with sequences set forth in SEQ ID NOs: 24 and 25); and 2004hzVH9 and 2004hzVK9 were combined with the human heavy chain constant region and the human light chain constant region, respectively, and then paired with each other to form a humanized antibody 2004hz99 (with sequences set forth in SEQ ID NOs: 26 and 27).

After codon optimization, gene synthesis was performed, and the synthesized gene fragment was cloned into an expression vector pcDNA3.4 (Life Technologies). After amplification of expression plasmids and plasmid extraction (Qiagen, EndoFree^{®} Plasmid Maxi Kit, Cat. No. 12362), dual plasmids were co-transfected into Expi293F (ThermoFisher Scientific, A14527) or CHO-K1 cells (ECACC catalogue no. 85051005), and the antibody was transiently transfected into cells and expressed according to the supplier's Expi293F or CHO-K1 expression system method. After the expression, the antibody was purified by a Protein A affinity chromatography column, and eluted with a citric acid buffer (pH 3.4), and an absorbance value at 280 nm was read using a NanoDrop instrument. The antibody was collected by dialysis for later use.

The results of antibody expression are shown in Table 2, which indicates that anti-ADM antibodies can be expressed by the method described above.

**Table 2. Expression levels of candidate antibodies**

| Antibody Name | Expression level (mg/L) |
|---|---|
| 2004hz00 | 71.7 |
| 2004hz97 | 260.0 |
| 2004hz99 | 215.0 |

### Example 3: Affinity Assay of Anti-ADM Antibodies

The affinity of antibodies 2004hz00, 2004hz97, and 2004hz99 for biotin-labeled human and mouse ADM (human ADM Cat. No. 894757, mouse ADM Cat. No. 894758, synthesized by GLBiochem) was determined by adopting Octet RED96e (Fortebio). The antigens and the antibodies were all diluted with 1× PBST (1× PBS: Sangon, B548117-0500; 0.02% Tween 20: sigma, P1379), the concentration of the antibody used was 100 nM, and the concentration of the antigen used was 2 µg/mL. The candidate antibody sample was added to a 96-well plate (Greiner bio-one, 655209) at 200 µL/well, the software parameters were set, the temperature was 30 °C, and the standard kinetic signal was collected at a frequency of 5.0 Hz; and an SA sensor (Fortebio, Cat. No. 18-5020) was pre-wetted with 1× PBST for 10 min and then tested on a machine.

Each cycle included the following steps: 1) immersing the sensor in a buffer for 60 s; 2) detecting whether the antigen non-specifically bound to the sensor; 3) regenerating the sensor with a 10 mM glycine solution at a pH of 1.7; 4) immersing the sensor in the buffer for 60 s; 5) immobilizing the antigen on the sensor for 10 s; 6) immersing the sensor in the buffer for 180 s; 7) binding the antigen to the antibody for 180 s; 8) dissociating the antigen and the antibody for 600 s; and 9) regenerating the sensor.

The equilibrium dissociation constants (KD) of the antibodies were calculated by measuring the association rates (Kon) and the dissociation rates(Koff) of the antigen-antibody in a 1:1 binding manner by adopting the Data Analysis 12.0 software from Fortebio. The results are shown in Tables 3 and 4, respectively. It can be seen from Tables 3 and 4 that the affinity of the humanized antibodies 2004hz97 and 2004hz99 for the human and mouse ADM was equivalent to that of the chimeric antibody 2004hz00.

**Table 3. Affinity of candidate antibodies for human ADM**

| Antibody Name | Response value | KD (M) | kon(1/Ms) | Koff (1/s) |
|---|---|---|---|---|
| 2004hz00 | 2.69 | 4.57E-10 | 4.41E+05 | 2.01E-04 |
| 2004hz97 | 2.39 | 5.46E-10 | 4.61E+05 | 2.52E-04 |
| 2004hz99 | 2.58 | 4.29E-10 | 4.68E+05 | 2.01E-04 |

**Table 4. Affinity of candidate antibodies for mouse ADM**

| Antibody Name | Response value | KD (M) | kon(1/Ms) | Koff (1/s) |
|---|---|---|---|---|
| 2004hz00 | 3.19 | 4.54E-10 | 4.32E+05 | 1.96E-04 |
| 2004hz97 | 3.00 | 4.89E-10 | 4.72E+05 | 2.31E-04 |
| 2004hz99 | 3.06 | 4.18E-10 | 4.65E+05 | 1.94E-04 |

### Example 4: Evaluation of Physicochemical Properties of Humanized Antibodies

The expression levels and affinity of the antibodies 2004hz97 and 2004hz99 were relatively ideal. As candidate molecules, the antibodies were further evaluated for physicochemical druggability, as specifically shown below.

### 4.1 SEC-HPLC purity analysis

The sample concentration was adjusted to 1 mg/mL, and the sample was centrifuged. The supernatant was transferred to a sample bottle, and the sample bottle was placed in an HPLC sample tray. The chromatographic conditions were set as follows: chromatographic column: TSK G3000SWxl; detection wavelength: 280 nm; column temperature: 25 °C; sample chamber temperature: 5 °C; and flow rate: 0.5 mL/min. After the chromatographic column was equilibrated by adopting a mobile phase (200 mM phosphate buffer, pH 6.8), the sample was injected for analysis, and the data were analyzed using chromatographic software. The peak area percentage of each peak was calculated by a peak area normalization method, and the higher the percentage, the higher the purity of the antibody.

### 4.2 HIC-HPLC analysis

The sample concentration was adjusted to 1 mg/mL, and the sample was centrifuged. The supernatant was collected for assay. The chromatographic conditions were set as follows: chromatographic column: MAbPac^{™}HIC-10; detection wavelength: 214 nm; column temperature: 30 °C; sample chamber temperature: 5 °C; and flow rate: 0.8 mL/min. Gradient elution was performed using mobile phase A (50 mM phosphate buffer/1 M ammonium sulfate, pH 7.0) and mobile phase B (50 mM phosphate buffer, pH 7.0), and the retention time of the main peak was recorded, with short peak appearance time indicating a strong hydrophilic property of the antibody.

### 4.3 Melting temperature (Tm) value analysis

According to the Protein Thermal Shift^{™} Starter Kit instructions, 13 µL of the test sample solution was added to a PCR tube, 5 µL of Protein Thermal shift TM Buffer was added, and 2 µL of a 10× staining solution was added to allow the reaction volume to be 20 µL. The resulting solution was uniformly mixed, and then the mixture was centrifuged at 12000 rpm for 5 min to remove bubbles. The detection sample was placed in a PCR instrument for sample analysis. The Tm value of the sample was recorded, and the higher the Tm value, the better the thermal stability of the antibody.

### 4.4 Isoelectric focusing (iCIEF) analysis

The sample solution was added to the following system which had been uniformly mixed: 70 µL of 1% methylcellulose (MC), 80 µL of 5 M urea, 8 µL of ampholyte Pharmalyte pH 3-10, and 2 µL each of pI markers 5.5 and 9.5. An appropriate volume of ultrapure water was supplemented to 200 µL, and the resulting solution was mixed uniformly. The mixture was centrifuged, and the supernatant was collected and injected for analysis. After the analysis was completed, the result file was imported into the ChromPerfect software for spectrum integration processing. The isoelectric point and percentage of each peak were calculated, and the charge isomer distribution of the candidate antibodies was analyzed.

### 4.5 nrCE-SDS

The sample was diluted to 4 mg/mL with ultrapure water, and 25 µL of the sample was collected. 75 µL of an SDS sample buffer and 5 µL of 0.25 mol. L-1 iodoacetamide (IAM) were added, and the resulting solution was mixed uniformly and heated at 70 °C for 10 min. 90 µL of supernatant was put into a sample bottle and analyzed on a machine. The detection was performed by adopting a Beckman PA800 Plus capillary electrophoresis system and an uncoated capillary (with a total length of 31 cm and an effective length of 21 cm). The detection conditions were as follows: separation voltage: 15 KV; capillary temperature: 25 °C; sample chamber temperature: 15 °C; and detection wavelength: 220 nm. The area percentage of the corrected peak of the main peak was calculated.

It can be seen from Table 5 that the humanized antibodies 2004hz97 and 2004hz99 had good physicochemical properties after one-step protein A purification.

**Table 5. Analysis results of physicochemical properties of candidate antibodies**

| Sample name | SEC-HPLC | | | cIEFIEF | | | | nrCE-SDS | Tm (°C) | HIC-HPLC (min) |
|---|---|---|---|---|---|---|---|---|---|---|
| | HMWS% | Main Peak% | LMWS% | pI | Acidic peak% | Main peak% | Basic peak% | Main peak% | | |
| 2004hz99 | 0.6 | 99.4 | 0 | 9 | 11.4 | 41.1 | 47.5 | 96.2 | 69.44 | 16.44 |
| 2004hz97 | 0.5 | 99.5 | 0 | 9 | 12.7 | 42.2 | 45.1 | 95.2 | 70.21 | 16.44 |

### Example 5: Panning of Fully Human Single-Chain Phage Antibody Library

150 µL of Streptavidin Magnetic Beads (Thermo fisher, Cat. No. 88817) were pre-bound to 2 mL of a fully human single-chain phage antibody library and incubated at room temperature for 90 min to remove non-specific binding. 10 µg of human ADM (Cat. No. NT-H-2, synthesized by GenScript) and 150 µL of Streptavidin Magnetic Beads were added to the library phage after background removal, incubated at room temperature for 15 min, and washed 14 times with PBST (0.05% Tween-20 in PBS) to wash away unbound phages. The phages specifically binding to antigens were eluted with 450 µL of 100 mM hydrochloric acid. 50 µL of 1 M Tris-HCl at a pH of 11 was added to neutralize and infect *Escherichia coli* SS320 in the logarithmic growth phase, and the phages were generated and purified for the next round of screening. The screening method was the same as the first round, and only the antigen amount was reduced to 4 µg. The enrichment condition of the phages enriched after two rounds of screening was identified by an enzyme-linked immunosorbent assay (ELISA), and the results show that the phages were significantly enriched after two rounds of panning. 10 µL of the phages eluted after two rounds of panning were serially 10000-fold diluted, and 90 µL of *Escherichia coli* SS320 in the logarithmic growth phase was added. The mixture was left to stand for infection for 30 min, and then a resistant plate was coated and cultured at 37 °C overnight. The next day, monoclonal strains were picked from the resistant plate and placed in a 96-well deep-well plate with ampicillin/IPTG/2YT added, and the plate was incubated at 37 °C overnight. The next day, the plate was centrifuged at 4000 g for 10 min, and the supernatant was collected. The binding ability of the monoclones was identified by the enzyme-linked immunosorbent assay (ELISA) to screen the monoclone 1F12 (with amino acid sequences of variable regions set forth in SEQ ID NOs: 28 and 35).

### Example 6: Effect of Anti-ADM Antibody on Biological Activity of ADM

The effect of the selected anti-ADM antibody on the biological activity of ADM was assayed in a human recombinant adrenomedullin receptor cAMP function assay (adrenomedullin bioassay).

The anti-ADM antibody was diluted to 1600 µg/mL with Stimulation Buffer 1 (Cisbio, 64SB1FDD) at a working concentration of 400 µg/mL, followed by 3-fold gradient dilution with Stimulation Buffer 1 (8 µL + 16 µL Stimulation Buffer 1), wherein the antagonist human ADM (22-52) (Alfa Aesar, Cat. No. 159899-65-7) was diluted to 12000 µg/mL at a working concentration of 3000 µg/mL. Subsequently, 2.5 µL of the anti-ADM antibody was added to the corresponding wells of an experimental plate. The human ADM JMB2004 YY-52 protein (GL Biochem, Cat. No. 196191) was diluted to 0.6 µg/mL with Stimulation Buffer 1, and 2.5 µL of the protein was added to the corresponding wells. That is, the working concentration of the human ADM JMB2004 YY-52 protein was 0.15 µg/mL, and then the experimental plate was incubated at room temperature for 60 min. CHO-K1 cells expressing human recombinant adrenomedullin receptors (hereinafter referred to as CHO-K1/CRLR/RAMP3, wherein the gene accession number of CRLR is U17473 and the gene accession number of RAMP3 is AJ001016) were digested and isolated with TrypLE Express (gibco, Cat. No. 12604-021), collected by centrifugation, and resuspended in Stimulation Buffer 1. The cell density was adjusted to 4 × 10⁶ cells/mL, and 5 µL of CHO-K1/CRLR/RAMP3 cells were added to each well (at 2 × 10⁴ cells/well). Subsequently, after the experimental plate was incubated in a cell incubator at 37 °C for 90 min, the cAMP content was detected using an HTRF kit (Cisbio, 62AM4PEB). That is, 5 µL of a cAMP-d2 reagent working solution was added to the experimental wells, and then 5 µL of a cAMP Eu-Cryptate antibody working solution was added. After the plate was incubated at room temperature for 1 h, the HTRF value was detected by a microplate reader.

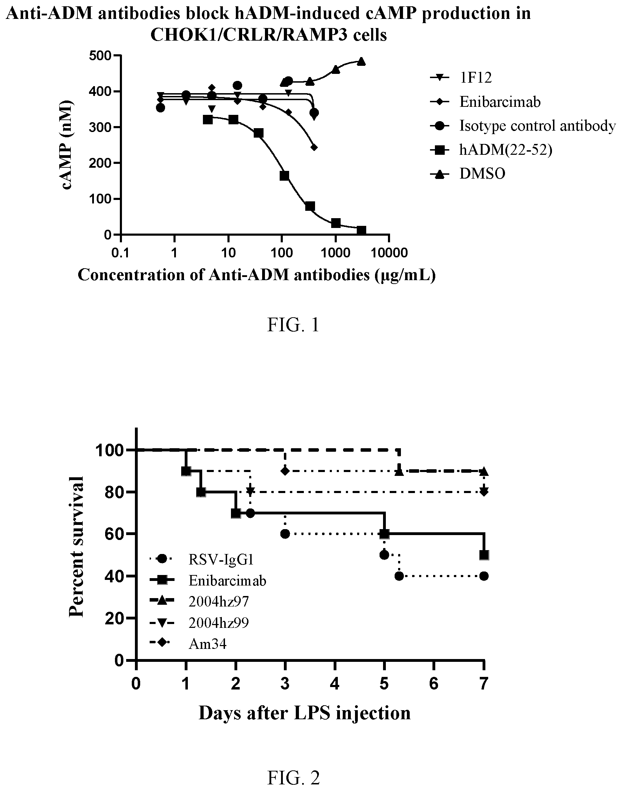

The experimental results are shown in FIG. 1, and it can be seen from FIG. 1 that the antibody 1F12 had no blocking activity.

### Example 7: Affinity Maturation Library Design and Construction of Fully Human Antibody 1F12

The affinity maturation was performed on the fully human antibody 1F12 obtained by screening to improve the affinity of the antibody for human ADM. At the same time, since the heavy chain antigen-binding determinant 2 (CDR_H2) of 1F12 contained a post-translational modification (PTM) site of NG, the site was mutated into QG by site-directed mutagenesis to remove deamidation and isomerization effects, and the mutated antibody was named 1F12 PTMΔ (with amino acid sequences of variable regions set forth in SEQ ID NOs: 29 and 35). The mutant 1F12 PTMΔ was used as a parent and numbered according to the Chothia scheme, and the CDRs were defined according to the Chothia. The amino acids at HCDR-3 and LCDR-3 sites were randomly mutated to construct a mutation library. NNK mutant primers were designed to amplify HCDR-3 and LCDR-3 mutation library gene fragments by the polymerase chain reaction (PCR). The amplified VH and VL gene fragments were recovered and electrotransferred into a *Saccharomyces cerevisiae* strain EBY100 (purchased from ATCC) together with yeast display plasmids. The VH and VL genes were inserted into the yeast display plasmids by homologous recombination of *Saccharomyces cerevisiae,* then a Fab mutation library of the antibody displayed on the surface of the yeast cell wall was realized, and the library was named JYYDL196-197. After the electrotransfer, the library JYYDL196-197 was cultured in 250 mL of an SD-Trp-Leu liquid medium (Clontech, Cat. No. 630316) at 30 °C overnight; 1.0 × 10⁹ yeast was taken and resuspended in 200 mL of a YPGP induction medium (2% galactose, 2% peptone, 1% yeast extract, 0.54% Na₂HPO₄, 0.86% NaH₂PO₄·H₂O), and the yeast was cultured at 20 °C for 24 h, and placed at 4 °C for later use. At the same time, the sequence of the parent 1F12 PTMΔ was displayed on the surface of the yeast and used as a parent control.

### Example 8: Affinity Maturation Library Screening and Monoclonal Identification of Fully Human Antibody 1F12

For the induced yeast solution of the JYYDL196-197 library, the OD₆₀₀ of the yeast solution was determined, and calculated according to 10D as the cell number of 1.0 × 10⁷. 1.0 × 10⁹ cells were taken, and a first round of enrichment was performed using a magnetic bead sorting system: the cells were washed once with 50 mL of 1× PBSA (1× PBS + 1% BSA) and centrifuged, and the supernatant was discarded; the cells were incubated with 5 mL of 1× PBSA containing 100 nM biotin-labeled human ADM (hADM-Biotin for short, Cat. No. NT-H-2, synthesized by GenScript) at room temperature for 30 min; and after washing, anti-biotin magnetic beads (miltenyi, Cat. No. 130-090-485) were added, and the resulting mixture was mixed uniformly and incubated for 10 min. The mixture was passed through a magnetic column (Quadro MACS Starting Kit), and the positive cells were collected. The positive cells were cultured and induced again, and then 3.0 × 10⁷ cells were taken for a second round of flow cytometry: 1 mL of 1× PBSA was used, the mixture was centrifuged, and the supernatant was discarded; the cells were incubated with 1 mL of 1× PBSA containing 10 nM hADM-Biotin and mouse anti-V5 antibody (Invitrogen, Cat. No. 2156578, 1:1000 dilution) for 30 min on ice; the mixture was centrifuged, the supernatant was discarded, 1 mL of 1× PBSA was added, and the mixture was washed once; 500 µL of 1× PBSA containing the fluorescent antibody (SA-PE, manufacturer: eBioscience, Cat. No. 12-4317-8, diluted in 1:200; goat anti-mouse-647, manufacturer: Invitrogen, Cat. No. A21235, diluted in 1:400) was added, and the mixture was incubated on ice in the dark for 20 min; and after washing, 2 mL of 1× PBSA was added to resuspend the cells, and the cell populations with both strong 647 fluorescence signal and PE fluorescence signal were collected by a flow cytometer. After the second round of flow cytometry, the cells were cultured and induced again, and then the method was the same as the method in the second round. 3.0 × 10⁷ cells were taken and incubated with 3 nM hADM-Biotin, and then the mixture was subjected to a third round of flow cytometry. After the sorting, some cells were applied on an SD-Trp-Leu solid medium (Clontech, Cat. No. 630317) plate, and the plate was cultured and left to stand at 30 °C for 3 days. For the third round of screening products of JYYDL196-197, 92 monoclones were picked for sequencing analysis. Finally, a yeast monoclonal colony with a unique sequence was obtained for flow staining analysis, and 1 × 10⁶ cells were selected for staining evaluation. According to the staining results of each clone in combination with the similarity of each clone sequence, the fully human antibody Ab2004.Am01 (with amino acid sequences of variable regions set forth in SEQ ID NOs: 30 and 35) was finally picked for expression.

### Example 9: Affinity Maturation Library Design and Construction of Ab2004.Am01

The affinity maturation was performed on Ab2004.Am01 to improve its affinity for human and mouse ADM. Referring to Example 7, the amino acids of HCDR-1, HCDR-2, LCDRL-1, LCDR-2, and LCDR-3 of Ab2004.Am01 were selected for mutation library construction, with the library number of JYYDL208-212. The library JYYDL208-212 was cultured and induced for later use. At the same time, the Fab sequence of Ab2004.Am01 was displayed on the surface of the yeast and used as a parent control in this round of affinity maturation.

### Example 10: Affinity Maturation Library Screening of Ab2004.Am01

For the induced yeast solution of JYYDL208-212 library, 1.0 × 10⁹ cells were taken, and a first round of enrichment was performed using a magnetic bead sorting system. After the magnetic bead screening, the positive cells were cultured and induced again, and 3.0 × 10⁷ cells were taken for a second round of flow cytometry. The cell populations with both strong 647 fluorescence signal and PE fluorescence signal were collected by a flow cytometer. The cell populations were cultured and induced, and then a third round of flow cytometry was performed. For the second round of screening products of JYYDL208-209, 3.0 × 10⁷ cells were taken for the third round of flow cytometry under the condition of 10 nM mADM-Biotin; and for JYYDL210-212, 3.0 × 10⁷ cells were taken for the third round of flow cytometry under the condition of 100 nM mADM-Biotin. After the second and third rounds of sorting, the cells were applied on an SD-Trp-Leu solid medium plate, and the plate was cultured and left to stand at 30 °C for 3 days.

### Example 11: Light and Heavy Chain Mutation Combinatorial Library Construction and Screening

For the second and third rounds of screening products of JYYDL208-209 and JYYDL211, several monoclones were picked to construct a light and heavy chain mutation combinatorial library JYYDL227.

For JYYDL227, 2.0 × 10⁷ cells were taken for a first round of flow cytometry with 3 nM hADM-Biotin and 1.2 nM mADM-Biotin, respectively; and after sorting, the cells were applied on an SD-Trp-Leu solid medium, and the medium was cultured and left to stand at 30 °C for 3 days.

For the first round of screening products of JYYDL227, 46 monoclones were picked for sequencing, and finally, a yeast monoclonal colony with a unique sequence was obtained for EC₅₀ identification by flow staining. 1 × 10⁵ cells were taken for staining evaluation: 1. the binding level of each clone to human ADM under different antigen concentrations was evaluated, and the EC₅₀ value of each clone to human ADM was calculated, wherein the smaller the value, the stronger the affinity; and 2. the binding level of each clone to mouse ADM under different antigen concentrations was evaluated, and similarly, the affinity of each clone for mouse ADM could be obtained. According to the staining results of the clones (as shown in Table 6) in combination with the similarity of the sequences of the clones, the monoclones expressing antibodies Ab2004.Am31 (with amino acid sequences of variable regions set forth in SEQ ID NOs: 31 and 36), Ab2004.Am32 (with amino acid sequences of variable regions set forth in SEQ ID NOs: 31 and 37), Ab2004.Am33 (with amino acid sequences of variable regions set forth in SEQ ID NOs: 32 and 36), Ab2004.Am34 (with amino acid sequences of variable regions set forth in SEQ ID NOs: 33 and 38), Ab2004.Am35 (with amino acid sequences of variable regions set forth in SEQ ID NOs: 31 and 38), Ab2004.Am36 (with amino acid sequences of variable regions set forth in SEQ ID NOs: 31 and 39), Ab2004.Am37 (with amino acid sequences of variable regions set forth in SEQ ID NOs: 34 and 38), Ab2004.Am38 (with amino acid sequences of variable regions set forth in SEQ ID NOs: 34 and 40), Ab2004.Am39 (with amino acid sequences of variable regions set forth in SEQ ID NOs: 33 and 41), and Ab2004.Am40 (with amino acid sequences of variable regions set forth in SEQ ID NOs: 31 and 42) were finally selected. The amino acid sequences of the Chothia-numbered CDRs of the candidate antibodies are shown in Table 7, and the amino acid sequences of the light and heavy chain variable regions of the candidate antibodies are shown in Table 8.

**Table 6. Flow staining results of yeast monoclonal colonies**

| Antibody Name | Human ADM EC₅₀ (nM) | Mouse ADM EC₅₀ (nM) |
|---|---|---|
| Ab2004.Am01 | 4.00 | 88.22 |
| Ab2004.Am31 | 0.45 | 1.66 |
| Ab2004.Am32 | 0.41 | 2.20 |
| Ab2004.Am33 | 0.79 | 15.04 |
| Ab2004.Am34 | 1.06 | 4.43 |
| Ab2004.Am35 | 1.47 | 2.26 |
| Ab2004.Am36 | 1.73 | 2.67 |
| Ab2004.Am37 | 0.88 | 6.74 |
| Ab2004.Am38 | 0.74 | 14.3 |
| Ab2004.Am39 | 0.86 | 4.23 |
| Ab2004.Am40 | 0.50 | 2.50 |

**Table 7. Chothia-numbered CDR sequences of candidate antibodies**

| | HCDR1 | HCDR2 | HCDR3 | LCDR1 | LCDR2 | LCDR3 |
|---|---|---|---|---|---|---|
| 1F12 | GYTFTSY (SEQ ID NO: 43) | SAYNGN (SEQ ID NO: 44) | EGRSGGSFDI (SEQ ID NO: 45) | RASQGISSYLA (SEQ ID NO:46) | DASNLET (SEQ ID NO:47) | QQYDNLPLT (SEQ ID NO:48) |
| IF12-PTM Δ | GYTFTSY (SEQ ID NO: 43) | SAYQGN (SEQ ID NO: 49) | EGRSGGSFDI (SEQ ID NO: 45) | RASQGISSYLA (SEQ ID NO:46) | DASNLET (SEQ ID NO:47) | QQYDNLPLT (SEQ ID NO:48) |
| Ab2004.Am01 | GYTFTSY (SEQ ID NO: 43) | SAYQGN (SEQ ID NO: 49) | EGRWGGSFNI (SEQ ID NO:50) | RASQGISSYLA (SEQ ID NO:46) | DASNLET (SEQ ID NO:47) | QQYDNLPLT (SEQ ID NO:48) |
| Ab2004.Am31 | GYTFTQY (SEQ ID NO: 51) | SAYQGN (SEQ ID NO: 49) | EGRWGGSFNI (SEQ ID NO:50) | RASEGISEYLA (SEQ ID NO:52) | DASNLET (SEQ ID NO:47) | QQYDNLPLT (SEQ ID NO:48) |
| Ab2004.Am32 | GYTFTQY (SEQ ID NO: 51) | SAYQGN (SEQ ID NO: 49) | EGRWGGSFNI (SEQ ID NO:50) | RAAEGIGSYLA (SEQ ID NO:53) | DASNLET (SEQ ID NO:47) | QQYDNLPLT (SEQ ID NO:48) |
| Ab2004.Am33 | GYTFTSY (SEQ ID NO: 43) | SPYSGN (SEQ ID NO: 54) | EGRWGGSFNI (SEQ ID NO:50) | RASEGISEYLA (SEQ ID NO:52) | DASNLET (SEQ ID NO:47) | QQYDNLPLT (SEQ ID NO:48) |
| Ab2004.Am34 | GYTFTHY (SEQ ID NO: 55) | SAYQGN (SEQ ID NO: 49) | EGRWGGSFNI (SEQ ID NO:50) | RASQGISSYLA (SEQ ID NO:46) | DVSILDA (SEQ ID NO:56) | QQYDNLPLT (SEQ ID NO:48) |
| Ab2004.Am35 | GYTFTQY (SEQ ID NO: 51) | SAYQGN (SEQ ID NO: 49) | EGRWGGSFNI (SEQ ID NO:50) | RASQGISSYLA (SEQ ID NO:46) | DVSILDA (SEQ ID NO:56) | QQYDNLPLT (SEQ ID NO:48) |
| Ab2004.Am36 | GYTFTQY (SEQ ID NO: 51) | SAYQGN (SEQ ID NO: 49) | EGRWGGSFNI (SEQ ID NO:50) | RASQGISSYLA (SEQ ID NO:46) | DASNVDT (SEQ ID NO:57) | QQYDNLPLT (SEQ ID NO:48) |
| Ab2004.Am37 | GYTFTSY (SEQ ID NO: 43) | SPYTGK (SEQ ID NO: 58) | EGRWGGSFNI (SEQ ID NO:50) | RASQGISSYLA (SEQ ID NO:46) | DVSILDA (SEQ ID NO:56) | QQYDNLPLT (SEQ ID NO:48) |
| Ab2004.Am38 | GYTFTSY (SEQ ID NO: 43) | SPYTGK (SEQ ID NO: 58) | EGRWGGSFNI (SEQ ID NO:50) | RASQGISSYLA (SEQ ID NO:46) | DTSDLDT (SEQ ID NO:59) | QQYDNLPLT (SEQ ID NO:48) |
| Ab2004.Am39 | GYTFTHY (SEQ ID NO: 55) | SAYQGN (SEQ ID NO: 49) | EGRWGGSFNI (SEQ ID NO:50) | RASQGISSYLA (SEQ ID NO:46) | DASNLET (SEQ ID NO:47) | QQYDDLDLT (SEQ ID NO:60) |
| Ab2004.Am40 | GYTFTQY | SAYQGN | EGRWGGSFNI | RASQGISSYLA | DASNLET | QQYDDLPLS |
| | (SEQ ID NO: 51) | (SEQ ID NO: 49) | (SEQ ID NO:50) | (SEQ ID NO:46) | (SEQ ID NO:47) | (SEQ ID NO:61) |

**Table 8. Variable region sequences of candidate antibodies**

| Antibody Name | VH | VL |
|---|---|---|
| 1F12 | SEQ ID NO: 28 | SEQ ID NO: 35 |
| 1F12-PTMΔ | SEQ ID NO:29 | SEQ ID NO: 35 |
| Ab2004.Am01 | SEQ ID NO: 30 | SEQ ID NO: 35 |
| Ab2004.Am31 | SEQ ID NO:31 | SEQ ID NO: 36 |
| Ab2004.Am32 | SEQ ID NO: 31 | SEQ ID NO: 37 |
| Ab2004.Am33 | SEQ ID NO: 32 | SEQ ID NO: 36 |
| Ab2004.Am34 | SEQ ID NO: 33 | SEQ ID NO: 38 |
| Ab2004.Am35 | SEQ ID NO: 31 | SEQ ID NO: 38 |
| Ab2004.Am36 | SEQ ID NO: 31 | SEQ ID NO: 39 |
| Ab2004.Am37 | SEQ ID NO: 34 | SEQ ID NO: 38 |
| Ab2004.Am38 | SEQ ID NO:34 | SEQ ID NO:40 |
| Ab2004.Am39 | SEQ ID NO: 33 | SEQ ID NO: 41 |
| Ab2004.Am40 | SEQ ID NO: 31 | SEQ ID NO: 42 |

### Example 12: Expression of Candidate Antibodies

Similar to Example 2.2, VH and VK sequences of each clone were combined with the human Kappa chain constant region (CL, with an amino acid sequence set forth in SEQ ID NO: 20) and the human IgG1 constant region (CH, with an amino acid sequence set forth in SEQ ID NO: 21) to form antibody light and heavy chains, which were loaded into the expression vector pcDNA3.4 (Life Technologies). The vector was transiently transfected into CHO cells, expressed, and purified by Nanjing GenScript Biotech Co., Ltd. Candidate antibodies with affinity maturation were finally obtained as shown in Table 9.

**Table 9. Expression and purification data of candidate antibodies with affinity maturation**

| Antibody Name | Theoretical isoelectric point | Extinction coefficient | Expression level (mg/L) |
|---|---|---|---|
| Ab2004.Am31 | 8.0 | 1.46 | 307 |
| Ab2004.Am32 | 8.1 | 1.46 | 227 |
| Ab2004.Am33 | 8.0 | 1.46 | 235 |
| Ab2004.Am34 | 8.2 | 1.46 | 298 |
| Ab2004.Am35 | 8.2 | 1.46 | 318 |
| Ab2004.Am36 | 8.2 | 1.46 | 221 |
| Ab2004.Am37 | 8.3 | 1.46 | 305 |
| Ab2004.Am38 | 8.2 | 1.46 | 343 |
| Ab2004.Am39 | 8 | 1.46 | 275 |
| Ab2004.Am40 | 8.1 | 1.46 | 226 |

### Example 13: Affinity Assay of Candidate Antibodies

Similar to Example 3, the affinity of the affinity-matured antibodies Ab2004.Am31-Ab2004.Am40 and the parent antibody Ab2004.Am01 for human and mouse ADM was further determined, respectively, and the results are shown in Table 10. It can be seen from Table 10 that the affinity of Ab2004.Am31, Ab2004.Am34, and Ab2004.Am39 for human ADM was greatly improved relative to the parent antibody Ab2004.Am01, and the affinity for mouse ADM was comparable to that of the positive control antibody Enibarcimab.

**Table 10. Affinity assay of candidate antibodies for human and mouse ADM**

| Antigen | Antibody No. | Response value | *K_{D}* (M) | kon (1/Ms) | koff (1/s) |
|---|---|---|---|---|---|
| Human ADM | Enibarcimab | 2.07 | 5.67E-10 | 6.73E+05 | 3.82E-04 |
| | Ab2004.Am01 | 2.34 | 7.66E-10 | 3.05E+05 | 2.33E-04 |
| | Ab2004.Am31 | 2.64 | 2.88E-10 | 4.91E+05 | 1.41E-04 |
| | Ab2004.Am32 | 2.53 | 3.40E-10 | 4.30E+05 | 1.46E-04 |
| | Ab2004.Am33 | 2.63 | 3.16E-10 | 6.76E+05 | 2.14E-04 |
| | Ab2004.Am34 | 2.61 | 1.54E-10 | 4.44E+05 | 6.81E-05 |
| | Ab2004.Am35 | 1.96 | 2.81E-10 | 4.23E+05 | 1.19E-04 |
| | Ab2004.Am36 | 2.70 | 2.91E-10 | 3.72E+05 | 1.08E-04 |
| | Ab2004.Am37 | 2.88 | 2.56E-10 | 5.87E+05 | 1.50E-04 |
| | Ab2004.Am38 | 2.83 | 2.55E-10 | 6.26E+05 | 1.60E-04 |
| | Ab2004.Am39 | 2.57 | 1.78E-10 | 4.68E+05 | 8.33E-05 |
| | Ab2004.Am40 | 2.66 | 3.52E-10 | 4.49E+05 | 1.58E-04 |
| Mouse ADM | Enibarcimab | 2.75 | 4.97E-10 | 5.97E+05 | 2.97E-04 |
| | Ab2004.Am01 | 2.35 | 2.21E-09 | 4.34E+05 | 9.56E-04 |
| | Ab2004.Am31 | 2.81 | 4.12E-10 | 5.37E+05 | 2.21E-04 |
| | Ab2004.Am32 | 2.76 | 4.74E-10 | 4.61E+05 | 2.19E-04 |
| | Ab2004.Am33 | 2.80 | 4.74E-10 | 7.69E+05 | 3.65E-04 |
| | Ab2004.Am34 | 2.78 | 4.51E-10 | 4.92E+05 | 2.22E-04 |
| | Ab2004.Am35 | 2.74 | 4.52E-10 | 4.56E+05 | 2.06E-04 |
| | Ab2004.Am36 | 2.75 | 5.18E-10 | 4.27E+05 | 2.21E-04 |
| | Ab2004.Am37 | 2.85 | 4.69E-10 | 7.03E+05 | 3.30E-04 |
| | Ab2004.Am38 | 2.88 | 4.67E-10 | 7.33E+05 | 3.42E-04 |
| | Ab2004.Am39 | 2.70 | 4.89E-10 | 5.43E+05 | 2.66E-04 |
| | Ab2004.Am40 | 2.65 | 4.92E-10 | 5.13E+05 | 2.53E-04 |

### Example 14: Evaluation of Physicochemical Properties of Candidate Antibodies

The expression levels and affinity of the candidate antibodies Ab2004.Am31, Ab2004.Am34, and Ab2004.Am39 were relatively ideal, and the candidate antibodies were further evaluated for physicochemical druggability. The results are summarized in Table 11. It can be seen from Table 11 that Ab2004.Am31, Ab2004.Am34, and Ab2004.Am39 all met the druggability criteria in terms of the purity, thermal stability, hydrophilic property, charge isomer, etc. The method referred to that in Example 4.

**Table 11. Analysis results of physicochemical properties of affinity-matured antibodies**

| Antibody Name | SEC-HPLC (%) | Tm (°C) | Peak appearance time (min) | iCIEF | | | | NR-CE-SDS (%) | |
|---|---|---|---|---|---|---|---|---|---|
| | | | | Isoelectric point (pI) | Acidic peak (%) | Main peak (%) | Basic peak (%) | Fragment | Main peak |
| Ab2004.Am31 | 99.6 | 86.9 | 13.3 | 8.4 | 18.1 | 71.2 | 10.7 | 7.5 | 92.5 |
| Ab2004.Am34 | 99.6 | 84.9 | 14.5 | 8.8 | 20.2 | 69.2 | 10.6 | 5.7 | 94.3 |
| Ab2004.Am39 | 99.2 | 84.0 | 13.3 | 8.4 | 16.3 | 72.3 | 11.5 | 6.5 | 93.5 |

### Example 15: Pharmacodynamic Study of ADM Antibodies in LPS-Induced C57BL/6J Mouse Sepsis Model

C57BL/6J mice (10-11 weeks old, male) were purchased from Beijing Vital River Laboratory Animal Technology Co., Ltd., 50 in total. The animals were randomly divided into the following five groups according to their body weight (n = 10): G1 group was a model group given isotype control RSV-IgG1 (2 mg/kg, IV, Single), G2 group was a positive control group given Enibarcimab (2 mg/kg, IV, Single), and G3, G4, and G5 groups were given the antibodies 2004hz97 (2 mg/kg, IV, Single), 2004hz99 (2 mg/kg, IV, Single), and Ab2004.Am34 (2 mg/kg, IV, Single) of the present disclosure, respectively. A single IV treatment with corresponding antibodies was given 5 min before sepsis was induced by LPS, and 5 min later, a single IP administration of 20 mg/kg LPS (*E. coli* 055:B5; Sigma) was performed to induce sepsis in the mice. After modeling, the death condition of the animals was observed twice a day for 7 consecutive days. The data were plotted by the GraphPad Prism 8 software, and were statistically analyzed by a Log-rank (Mantel-Cox) test method. The survival rates of the animals in these groups after 7 days of LPS treatment were, respectively as follows: RSV-IgG1 (40%), Enibarcimab (50%, P = 0.6713 vs RSV-IgG1), 2004hz97 (90%, P < 0.05 vs RSV-IgG1), 2004hz99 (80%, P = 0.1001 vs RSV-IgG1), Am34 (80%, P < 0.05 vs RSV-IgG1). The results show that after the treatment with the antibodies of the present disclosure, the survival rate of the animals was significantly increased and the symptoms of sepsis were significantly improved (see Table 12 and FIG. 2).

**Table 12. Survival rates of mice with LPS-induced sepsis affected by candidate antibodies**

| Drug | Number of animals | Dose of drug (mg/kg) | Survival rate after 7 days (%) |
|---|---|---|---|
| RSV-IgG1 | 10 | 2 | 40 |
| Enibarcimab | 10 | 2 | 50 |
| 2004hz97 | 10 | 2 | 90 |
| 2004hz99 | 10 | 2 | 80 |
| Ab2004.Am34 | 10 | 2 | 80 |

The foregoing is the preferred example of the present disclosure, and the present disclosure is not intended to be limited to the content disclosed in the example and the accompanying drawings. All equivalents and modifications obtained without departing from the spirit disclosed in the present disclosure shall fall within the protection scope of the present disclosure.

### SEQUENCE LISTING

| SEQ ID NO: | Sequence description | Sequence |
|---|---|---|
| 1 | hADM 1-16AA | YRQSMNNFQGLRSFGC |
| 2 | hADM 1-21AA | YRQSMNNFQGLRSFGCRFGTC |
| 3 | mADM 1-19AA | YRQSMNQGSRSNGCRFGTC |
| 4 | Human ADM | YRQSMNNFQGLRSFGCRFGTCTVQKLAHQIYQFTDKDKDNVAPRSKISP QGY YRQSMNQGSRSNGCRFGTCTFQKLAHQIYQLTDKDKDGMAPRNKISPQ GY |
| 5 | Mouse ADM | YRQSMNQGSRSNGCRFGTCTFQKLAHQIYQLTDKDKDGMAPRNKISPQ GY |
| 6 | VH primer | GGACAGGGMTCCAKAGTTCC |
| 7 | VK primer | GCACACGACTGAGGCACCTCCAGATGTT |
| 8 | polyC primer | AAGCAGTGGTATCAACGCAGAGTCATCCCCCCCCCCCCCCCC |
| 9 | 40E12 VH | QIQLVQSGPELRKPGETVKISCKASGYAFTTFGMSWIKQAPGQGLKWM GWINTYSRVPKYTDDFKGRFAFSLEISATTAYLLINNLKNGDTATYFCA RGYGGEGGLGFSGQGTTLTVSS |
| 10 | 40E12VK | QIQLVQSGPELRKPGETVKISCKASGYAFTTFGMSWIKQAPGQGLKWM GWINTYSRVPKYTDDFKGRFAFSLEISATTAYLLINNLKNGDTATYFCA RGYGGEGGLGFSGQGTTLTVSS DVLMTQTPFSLPVSLGDQASISCRSSQSIIDSDGNTYLEWYLQKPGQSPK LLIYKVSNRFSGVPDRFSGSGSGTDFTLRISRVEAEDLGVYYCFQGSHFP YTFAGGTKLELK |
| 11 | 40E12 HCDR1 | GYAFTTF |
| 12 | 40E12 HCDR2 | NTYSRV |
| 13 | 40E12 HCDR3 | GYGGEGGLGF |
| 14 | 40E12 LCDR1 | RSSQSIIDSDGNTYLE |
| 15 | 40E12 LCDR2 | KVSNRFS |
| 16 | 40E12 LCDR3 | FQGSHFPYT |
| 17 | 2004hzVH9 | QIQLVQSGSELKKPGASVKVSCKASGYAFTTFGMSWIRQAPGQGLEWM GWINTYSRVPKYTQGFTGRFVFSLDISVTTAYLQISSLKAEDTAVYFCAR GYGGEGGLGFSGQGTLVTVSS |
| 18 | 2004hzVK7 | DVVMTQSPLSLPVTLGQPASISCRSSQSIIDSDGNTYLEWYQQRPGQSPR LLIYKVSNRFSGVPDRFSGSGSGTDFTLKISRVEAEDFGVYYCFQGSHFP YTFGQGTKLEIK |
| 19 | 2004hzVK9 | DVLMTQSPLSLPVTLGQPASISCRSSQSIIDSDGNTYLEWYQQRPGQSPR LLIYKVSNRFSGVPDRFSGSGSGTDFTLKISRVEAEDFGVYYCFQGSHFP YTFAQGTKLEIK |
| 20 | Human light chain constant region CL | |
| 21 | Human heavy chain constant region CH | |
| 22 | 2004hz00 LC | DVLMTQTPFSLPVSLGDQASISCRSSQSIIDSDGNTYLEWYLQKPGQSPK LLIYKVSNRFSGVPDRFSGSGSGTDFTLRISRVEAEDLGVYYCFQGSHFP YTFAGGTKLELKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 23 | 2004hz00 HC | QIQLVQSGPELRKPGETVKISCKASGYAFTTFGMSWIKQAPGQGLKWM GWINTYSRVPKYTDDFKGRFAFSLEISATTAYLLINNLKNGDTATYFCA RGYGGEGGLGFSGQGTTLTVSSASTKGPSVFPLAPSSKSTSGGTAALGC LVKDYFPEPVTVSWNSGALTSGVHTFPAVLQSSGLYSLSSVVTVPSSSL GTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISK AKGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNH |
| | | YTQKSLSLSPGK |
| 24 | 2004hz97 LC | DVVMTQSPLSLPVTLGQPASISCRSSQSIIDSDGNTYLEWYQQRPGQSPR LLIYKVSNRFSGVPDRFSGSGSGTDFTLKISRVEAEDFGVYYCFQGSHFP YTFGQGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 25 | 2004hz97 HC | QIQLVQSGSELKKPGASVKVSCKASGYAFTTFGMSWIRQAPGQGLEWM GWINTYSRVPKYTQGFTGRFVFSLDISVTTAYLQISSLKAEDTAVYFCAR GYGGEGGLGFSGQGTLVTVSSASTKGPSVFPLAPSSKSTSGGTAALGCL VKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAK GQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 26 | 2004hz99 LC | DVLMTQSPLSLPVTLGQPASISCRSSQSIIDSDGNTYLEWYQQRPGQSPR LLIYKVSNRFSGVPDRFSGSGSGTDFTLKISRVEAEDFGVYYCFQGSHFP YTFAQGTKLEIKRTVAAPSVFIFPPSDEQLKSGTASVVCLLNNFYPREAK VQWKVDNALQSGNSQESVTEQDSKDSTYSLSSTLTLSKADYEKHKVYA CEVTHQGLSSPVTKSFNRGEC |
| 27 | 2004hz99 HC | QIQLVQSGSELKKPGASVKVSCKASGYAFTTFGMSWIRQAPGQGLEWM GWINTYSRVPKYTQGFTGRFVFSLDISVTTAYLQISSLKAEDTAVYFCAR GYGGEGGLGFSGQGTLVTVSSASTKGPSVFPLAPSSKSTSGGTAALGCL VKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAK GQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYT QKSLSLSPGK |
| 28 | 1F12 VH | QVQLVQSGAEVKKPGASVKVSCKASGYTFTSYGISWVRQAPGQGLEW MGWISAYNGNTNYAQKLQGRVTMTRDTSTSTAYMELRSLRSDDTAVY YCAREGRSGGSFDIWGQGTMVTVSS |
| 29 | 1F12-PTMΔ VH | QVQLVQSGAEVKKPGASVKVSCKASGYTFTSYGISWVRQAPGQGLEW MGWISAYQGNTNYAQKLQGRVTMTRDTSTSTAYMELRSLRSDDTAVY YCAREGRSGGSFDIWGQGTMVTVSS |
| 30 | Ab2004.Am01 VH | QVQLVQSGAEVKKPGASVKVSCKASGYTFTSYGISWVRQAPGQGLEW MGWISAYQGNTNYAQKLQGRVTMTRDTSTSTAYMELRSLRSDDTAVY YCAREGRWGGSFNIWGQGTMVTVSS |
| 31 | Ab2004.Am31 VH | QVQLVQSGAEVKKPGASVKVSCKASGYTFTQYGIAWVRQAPGQGLEW MGWISAYQGNTNYAQKLQGRVTMTRDTSTSTAYMELRSLRSDDTAVY YCAREGRWGGSFNIWGQGTMVTVSS |
| | Ab2004.Am32 VH | |
| | Ab2004.Am35 VH | |
| | Ab2004.Am36 VH | |
| | Ab2004.Am40 VH | |
| 32 | Ab2004.Am33 VH | QVQLVQSGAEVKKPGASVKVSCKASGYTFTSYGISWVRQAPGQGLEW MGWISPYSGNTNYAQKLQGRVTMTRDTSTSTAYMELRSLRSDDTAVY YCAREGRWGGSFNIWGQGTMVTVSS |
| 33 | Ab2004.Am34 VH | QVQLVQSGAEVKKPGASVKVSCKASGYTFTHYGIAWVRQAPGQGLEW MGWISAYQGNTNYAQKLQGRVTMTRDTSTSTAYMELRSLRSDDTAVY YCAREGRWGGSFNIWGQGTMVTVSS |
| | Ab2004.Am39 VH | |
| 34 | Ab2004.Am37 VH | QVQLVQSGAEVKKPGASVKVSCKASGYTFTSYGISWVRQAPGQGLEW MGWISPYTGKTNYAQKLQGRVTMTRDTSTSTAYMELRSLRSDDTAVY YCAREGRWGGSFNIWGQGTMVTVSS |
| | Ab2004.Am38 VH | |
| 35 | 1F12 VL | DIQMTQSPSSLSASVGDRVTITCRASQGISSYLAWYQQKPGKAPKLLIYD ASNLETGVPSRFSGSGSGTDFTFTISSLQPEDIATYYCQQYDNLPLTFGG GTKVEIK |
| | 1F12-PTMΔ VL | |
| | Ab2004.Am01 VL | |
| 36 | Ab2004.Am31 VL | DIQMTQSPSSLSASVGDRVTITCRASEGISEYLAWYQQKPGKAPKLLIYD ASNLETGVPSRFSGSGSGTDFTFTISSLQPEDIATYYCQQYDNLPLTFGG GTKVEIK |
| | Ab2004.Am33 VL | |
| 37 | Ab2004.Am32 VL | DIQMTQSPSSLSASVGDRVTITCRAAEGIGSYLAWYQQKPGKAPKLLIY DASNLETGVPSRFSGSGSGTDFTFTISSLQPEDIATYYCQQYDNLPLTFG GGTKVEIK |
| 38 | Ab2004.Am34 VL | DIQMTQSPSSLSASVGDRVTITCRASQGISSYLAWYQQKPGKAPKLLIYD VSILDAGVPSRFSGSGSGTDFTFTISSLQPEDIATYYCQQYDNLPLTFGGG TKVEIK |
| | Ab2004.Am35 VL | |
| | Ab2004.Am37 VL | |
| 39 | Ab2004.Am36 VL | DIQMTQSPSSLSASVGDRVTITCRASQGISSYLAWYQQKPGKAPKLLIYD ASNVDTGVPSRFSGSGSGTDFTFTISSLQPEDIATYYCQQYDNLPLTFGG GTKVEIK |
| 40 | Ab2004.Am38 VL | DIQMTQSPSSLSASVGDRVTITCRASQGISSYLAWYQQKPGKAPKLLIYD TSDLDTGVPSRFSGSGSGTDFTFTISSLQPEDIATYYCQQYDNLPLTFGG GTKVEIK |
| 41 | Ab2004.Am39 VL | DIQMTQSPSSLSASVGDRVTITCRASQGISSYLAWYQQKPGKAPKLLIYD ASNLETGVPSRFSGSGSGTDFTFTISSLQPEDIATYYCQQYDDLDLTFGG GTKVEIK |
| 42 | Ab2004.Am40 VL | DIQMTQSPSSLSASVGDRVTITCRASQGISSYLAWYQQKPGKAPKLLIYD ASNLETGVPSRFSGSGSGTDFTFTISSLQPEDIATYYCQQYDDLPLSFGGG TKVEIK |
| 43 | 1F12 HCDR1 | GYTFTSY |
| 44 | 1F12 HCDR2 | SAYNGN |
| 45 | 1F12 HCDR3 | EGRSGGSFDI |
| 46 | 1F12 LCDR1 | RASQGISSYLA |
| 47 | 1F12 LCDR2 | DASNLET |
| 48 | 1F12 LCDR3 | QQYDNLPLT |
| 49 | 1F12-PTMΔ HCDR2 | SAYQGN |
| 50 | Ab2004.Am01 HCDR3 | EGRWGGSFNI |
| 51 | Ab2004.Am31 HCDR1 | GYTFTQY |
| 52 | Ab2004.Am31 LCDR1 | RASEGISEYLA |
| 53 | Ab2004.Am32 LCDR1 | RAAEGIGSYLA |
| 54 | Ab2004.Am33 HCDR2 | SPYSGN |
| 55 | Ab2004.Am34 HCDR1 | GYTFTHY |
| 56 | Ab2004.Am34 LCDR2 | DVSILDA |
| 57 | Ab2004.Am36 LCDR2 | DASNVDT |
| 58 | Ab2004.Am37 HCDR2 | SPYTGK |
| 59 | Ab2004.Am38 LCDR2 | DTSDLDT |
| 60 | Ab2004.Am39 LCDR3 | QQYDDLDLT |
| 61 | Ab2004.Am40 LCDR3 | QQYDDLPLS |

## Claims

1. An anti-adrenomedullin (anti-ADM) monoclonal antibody or a fragment thereof, wherein the monoclonal antibody or the fragment specifically binds to a sequence of amino acids from position 1 to position 21 at the N-terminus of human adrenomedullin (ADM), the sequence of the amino acids from position 1 to position 21 at the N-terminus of the human ADM is set forth in SEQ ID NO: 2, and the monoclonal antibody or the fragment exhibits an affinity for ADM with a KD value of less than 10⁻¹⁰ M.

2. The anti-adrenomedullin monoclonal antibody or the fragment thereof according to claim **1,** wherein the fragment comprises a Fab, a Fab', an F(ab)2, an Fv fragment, an F(ab')2, a scFv, a di-scFv, a VHH, and/or a dAb.

3. The anti-adrenomedullin monoclonal antibody or the fragment thereof according to claim 1 or 2, wherein:
(a) a heavy chain variable region of the monoclonal antibody comprises the following CDR sequences:
(i) GYAFTTF (set forth in SEQ ID NO: 11),
(ii) NTYSRV (set forth in SEQ ID NO: 12), and
(iii) GYGGEGGLGF (set forth in SEQ ID NO: 13); and
(b) a light chain variable region of the monoclonal antibody comprises the following CDR sequences:
(i) RSSQSIIDSDGNTYLE (set forth in SEQ ID NO: 14),
(ii) KVSNRFS (set forth in SEQ ID NO: 15), and
(iii) FQGSHFPYT (set forth in SEQ ID NO: 16).

4. The anti-adrenomedullin monoclonal antibody or the fragment thereof according to claim 3, wherein the monoclonal antibody comprises a heavy chain variable region sequence set forth in any one of SEQ ID NOs: 9 and 17, and the monoclonal antibody comprises a light chain variable region sequence set forth in any one of SEQ ID NOs: 10, 18, and 19, wherein the monoclonal antibody preferably comprises the heavy chain variable region sequence set forth in SEQ ID NO: 9 and the light chain variable region sequence set forth in SEQ ID NO: 10, the monoclonal monomer preferably comprises the heavy chain variable region sequence set forth in SEQ ID NO: 17 and the light chain variable region sequence set forth in SEQ ID NO: 18, and the monoclonal monomer preferably comprises the heavy chain variable region sequence set forth in SEQ ID NO: 17 and the light chain variable region sequence set forth in SEQ ID NO: 19.

5. The anti-adrenomedullin monoclonal antibody or the fragment thereof according to claim 4, wherein the monoclonal antibody comprises a heavy chain sequence set forth in SEQ ID NO: 23 and a light chain sequence set forth in SEQ ID NO: 22.

6. The anti-adrenomedullin monoclonal antibody or the fragment thereof according to claim 4, wherein the monoclonal antibody comprises a heavy chain sequence set forth in SEQ ID NO: 25 and a light chain sequence set forth in SEQ ID NO: 24.

7. The anti-adrenomedullin monoclonal antibody or the fragment thereof according to claim 4, wherein the monoclonal antibody comprises a heavy chain sequence set forth in SEQ ID NO: 27 and a light chain sequence set forth in SEQ ID NO: 26.

8. The anti-adrenomedullin monoclonal antibody or the fragment thereof according to claim 1 or 2, wherein:
(a) a heavy chain variable region of the monoclonal antibody comprises the following CDR sequences:
(i) GYTFTX₁Y, wherein X₁ is selected from S, Q, and H,
(ii) SX₂YX₃GX₄, wherein X₂ is selected from A and P, X₃ is selected from N, Q, S, and T, and X₄ is selected from N and K, and
(iii) EGRX₅GGSFX₆I, wherein X₅ is selected from S and W, and X₆ is selected from D and N; and
(b) a light chain variable region of the monoclonal antibody comprises the following CDR sequences:
(i) RAX₇X₈GIX₉X₁₀YLA, wherein X₇ is selected from S and A, X₈ is selected from Q and E, X₉ is selected from S and G, and X₁₀ is selected from S and E,
(ii) DX₁₁SX₁₂X₁₃X₁₄X₁₅, wherein X₁₁ is selected from A, V, and T, X₁₂ is selected from N, I, and D, X₁₃ is selected from L and V, X₁₄ is selected from E and D, and X₁₅ is selected from T and A, and
(iii) QQYDX₁₆LX₁₇LX₁₈, wherein X₁₆ is selected from N and D, X₁₇ is selected from P and D, and X₁₈ is selected from T and S.

9. The anti-adrenomedullin monoclonal antibody or the fragment thereof according to claim 8, wherein:
(a) the heavy chain variable region of the monoclonal antibody comprises sequences of HCDR1, HCDR2, and HCDR3, wherein the HCDR1 comprises an amino acid sequence set forth in any one of SEQ ID NOs: 43, 51, and 55, the HCDR2 comprises an amino acid sequence set forth in any one of SEQ ID NOs: 44, 49, 54, and 58, and the HCDR3 comprises an amino acid sequence set forth in any one of SEQ ID NOs: 45 and 50, and
(b) the light chain variable region of the monoclonal antibody comprises sequences of LCDR1, LCDR2, and LCDR3, wherein the LCDR1 comprises an amino acid sequence set forth in any one of SEQ ID NOs: 46, 52, and 53, the LCDR2 comprises an amino acid sequence set forth in any one of SEQ ID NOs: 47, 56, 57, and 59, and the LCDR3 comprises an amino acid sequence set forth in any one of SEQ ID NOs: 48, 60, and 61.

10. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SAYNGN (set forth in SEQ ID NO: 44), and
(iii) EGRSGGSFDI (set forth in SEQ ID NO: 45); and
(b) the light chain variable region of the monoclonal antibody comprises the following CDR sequences:
(i) RASQGISSYLA (set forth in SEQ ID NO: 46),
(ii) DASNLET (set forth in SEQ ID NO: 47), and
(iii) QQYDNLPLT (set forth in SEQ ID NO: 48).

11. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SAYQGN (set forth in SEQ ID NO: 49), and
(iii) EGRSGGSFDI (set forth in SEQ ID NO: 45); and
(b) the light chain variable region of the monoclonal antibody comprises the following CDR sequences:
(i) RASQGISSYLA (set forth in SEQ ID NO: 46),
(ii) DASNLET (set forth in SEQ ID NO: 47), and
(iii) QQYDNLPLT (set forth in SEQ ID NO: 48).

12. The anti-adrenomedullin monoclonal antibody or the fragment thereof according to any one of claims 8 to 11, wherein the monoclonal antibody comprises a heavy chain variable region sequence set forth in SEQ ID NO: 28 or 29, and the monoclonal antibody comprises a light chain variable region sequence set forth in SEQ ID NO: 35, wherein the monoclonal antibody preferably comprises the heavy chain variable region sequence set forth in SEQ ID NO: 28 and the light chain variable region sequence set forth in SEQ ID NO: 35, and the monoclonal monomer preferably comprises the heavy chain variable region sequence set forth in SEQ ID NO: 29 and the light chain variable region sequence set forth in SEQ ID NO: 35.

13. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DASNLET (set forth in SEQ ID NO: 47), and
(iii) QQYDNLPLT (set forth in SEQ ID NO: 48).

14. The anti-adrenomedullin monoclonal antibody or the fragment thereof according to claim 9, wherein:
(a) the heavy chain variable region of the monoclonal antibody comprises the following CDR sequences:
(i) GYTFTQY (set forth in SEQ ID NO: 51),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASEGISEYLA (set forth in SEQ ID NO: 52),
(ii) DASNLET (set forth in SEQ ID NO: 47), and
(iii) QQYDNLPLT (set forth in SEQ ID NO: 48).

15. The anti-adrenomedullin monoclonal antibody or the fragment thereof according to claim 9, wherein:
(a) the heavy chain variable region of the monoclonal antibody comprises the following CDR sequences:
(i) GYTFTQY (set forth in SEQ ID NO: 51),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RAAEGIGSYLA (set forth in SEQ ID NO: 53),
(ii) DASNLET (set forth in SEQ ID NO: 47), and
(iii) QQYDNLPLT (set forth in SEQ ID NO: 48).

16. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SPYSGN (set forth in SEQ ID NO: 54), and
(iii) EGRWGGSFNI (set forth in SEQ ID NO: 50); and
(b) the light chain variable region of the monoclonal antibody comprises the following CDR sequences:
(i) RASEGISEYLA (set forth in SEQ ID NO: 52),
(ii) DASNLET (set forth in SEQ ID NO: 47), and
(iii) QQYDNLPLT (set forth in SEQ ID NO: 48).

17. The anti-adrenomedullin monoclonal antibody or the fragment thereof according to claim 9, wherein:
(a) the heavy chain variable region of the monoclonal antibody comprises the following CDR sequences:
(i) GYTFTHY (set forth in SEQ ID NO: 55),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DVSILDA (set forth in SEQ ID NO: 56), and
(iii) QQYDNLPLT (set forth in SEQ ID NO: 48).

18. The anti-adrenomedullin monoclonal antibody or the fragment thereof according to claim 9, wherein:
(a) the heavy chain variable region of the monoclonal antibody comprises the following CDR sequences:
(i) GYTFTQY (set forth in SEQ ID NO: 51),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DVSILDA (set forth in SEQ ID NO: 56), and
(iii) QQYDNLPLT (set forth in SEQ ID NO: 48).

19. The anti-adrenomedullin monoclonal antibody or the fragment thereof according to claim 9, wherein:
(a) the heavy chain variable region of the monoclonal antibody comprises the following CDR sequences:
(i) GYTFTQY (set forth in SEQ ID NO: 51),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DASNVDT (set forth in SEQ ID NO: 57), and
(iii) QQYDNLPLT (set forth in SEQ ID NO: 48).

20. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SPYTGK (set forth in SEQ ID NO: 58), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DVSILDA (set forth in SEQ ID NO: 56), and
(iii) QQYDNLPLT (set forth in SEQ ID NO: 48).

21. The anti-adrenomedullin monoclonal antibody or the fragment thereof according to claim 9, wherein:
(a) the heavy chain variable region of the monoclonal antibody comprises the following CDR sequences:
(i) GYTFTSY (set forth in SEQ ID NO: 43),
(ii) SPYTGK (set forth in SEQ ID NO: 58), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DTSDLDT (set forth in SEQ ID NO: 59), and
(iii) QQYDNLPLT (set forth in SEQ ID NO: 48).

22. The anti-adrenomedullin monoclonal antibody or the fragment thereof according to claim 9, wherein:
(a) the heavy chain variable region of the monoclonal antibody comprises the following CDR sequences:
(i) GYTFTHY (set forth in SEQ ID NO: 55),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DASNLET (set forth in SEQ ID NO: 47), and
(iii) QQYDDLDLT (set forth in SEQ ID NO: 60).

23. The anti-adrenomedullin monoclonal antibody or the fragment thereof according to claim 9, wherein:
(a) the heavy chain variable region of the monoclonal antibody comprises the following CDR sequences:
(i) GYTFTQY (set forth in SEQ ID NO: 51),
(ii) SAYQGN (set forth in SEQ ID NO: 49), and
(iii) EGRWGGSFNI (set forth in SEQ ID NO: 50); and
(b) the light chain variable region of the monoclonal antibody comprises the following CDR sequences:
(i) RASQGISSYLA (set forth in SEQ ID NO: 46),
(ii) DASNLET (set forth in SEQ ID NO: 47), and
(iii) QQYDDLPLS (set forth in SEQ ID NO: 61).

24. The anti-adrenomedullin monoclonal antibody or the fragment thereof according to any one of claims 8 to 9 and 13 to 23, wherein the monoclonal antibody comprises a heavy chain variable region sequence set forth in any one of SEQ ID NOs: 30 to 34, and the monoclonal antibody comprises a light chain variable region sequence set forth in any one of SEQ ID NOs: 35 to 42, wherein the monoclonal antibody preferably comprises the heavy chain variable region sequence set forth in SEQ ID NO: 30 and the light chain variable region sequence set forth in SEQ ID NO: 35, the monoclonal monomer preferably comprises the heavy chain variable region sequence set forth in SEQ ID NO: 31 and the light chain variable region sequence set forth in SEQ ID NO: 36, the monoclonal monomer preferably comprises the heavy chain variable region sequence set forth in SEQ ID NO: 31 and the light chain variable region sequence set forth in SEQ ID NO: 37, the monoclonal antibody preferably comprises the heavy chain variable region sequence set forth in SEQ ID NO: 32 and the light chain variable region set forth in SEQ ID NO: 36, the monoclonal monomer preferably comprises the heavy chain variable region sequence set forth in SEQ ID NO: 33 and the light chain variable region sequence set forth in SEQ ID NO: 38, the monoclonal monomer preferably comprises the heavy chain variable region sequence set forth in SEQ ID NO: 31 and the light chain variable region sequence set forth in SEQ ID NO: 38, the monoclonal antibody preferably comprises the heavy chain variable region sequence set forth in SEQ ID NO: 31 and the light chain variable region sequence set forth in SEQ ID NO: 39, the monoclonal monomer preferably comprises the heavy chain variable region sequence set forth in SEQ ID NO: 34 and the light chain variable region sequence set forth in SEQ ID NO: 38, the monoclonal monomer preferably comprises the heavy chain variable region sequence set forth in SEQ ID NO: 34 and the light chain variable region sequence set forth in SEQ ID NO: 40, the monoclonal antibody preferably comprises the heavy chain variable region sequence set forth in SEQ ID NO: 33 and the light chain variable region sequence set forth in SEQ ID NO: 41, and the monoclonal monomer preferably comprises the heavy chain variable region sequence set forth in SEQ ID NO: 31 and the light chain variable region sequence set forth in SEQ ID NO: 42.

25. A nucleotide sequence encoding the anti-adrenomedullin monoclonal antibody or the fragment thereof according to any one of claims 1 to 24.

26. An expression vector, comprising the nucleotide sequence according to claim 25, wherein preferably, the expression vector is pcDNA3.4.

27. A host cell, comprising the nucleotide sequence according to claim 25 or the expression vector according to claim 26, wherein preferably, the host cell is a CHO cell.

28. A pharmaceutical composition, a detection reagent, or a kit of parts, comprising the anti-adrenomedullin monoclonal antibody or the fragment thereof according to any one of claims 1 to 24.

29. Use of the anti-adrenomedullin monoclonal antibody or the fragment thereof according to any one of claims 1 to 24 for manufacturing a medicament for the maintenance of endothelial cell integrity and the enhancement of a barrier function.

30. Use of the anti-adrenomedullin monoclonal antibody or the fragment thereof according to any one of claims 1 to 24 for manufacturing a medicament for controlling vasodilation and lowering blood pressure.

31. Use of the anti-adrenomedullin monoclonal antibody or the fragment thereof according to any one of claims 1 to 24 for manufacturing a medicament for the treatment or prevention of septic shock or traumatic injury, especially an advanced stage of sepsis.

32. Use of the anti-adrenomedullin monoclonal antibody or the fragment thereof according to any one of claims 1 to 24 for manufacturing a medicament for the reduction of death risk in a patient with a chronic or acute disease or an acute condition, wherein the chronic or acute disease or the acute condition is selected from a severe infection such as meningitis, systemic inflammatory response syndrome (SIRS), and septicemia; other diseases such as diabetes, cancers, acute and chronic vascular diseases (such as heart failure, myocardial infarction, stroke, and atherosclerosis), and edema; and shock such as septic shock, organ dysfunction such as renal dysfunction and liver dysfunction, burns, surgery, trauma, and poisoning.

33. Use of the anti-adrenomedullin monoclonal antibody or the fragment thereof according to any one of claims 1 to 24 for manufacturing a medicament for the stabilization of circulation, especially systemic circulation, in a patient, wherein the patient suffers from a chronic or acute disease or an acute condition, and the chronic or acute disease or the acute condition is selected from a severe infection such as meningitis, systemic inflammatory response syndrome (SIRS), and septicemia; other diseases such as diabetes, cancers, acute and chronic vascular diseases (such as heart failure, myocardial infarction, stroke, and atherosclerosis), and edema; and shock such as septic shock, organ dysfunction such as renal dysfunction and liver dysfunction, burns, surgery, trauma, and poisoning.

34. The use according to claims 29 to 33, wherein the anti-adrenomedullin monoclonal antibody or the fragment thereof is used in combination with another medicament.

35. The use according to claim 34, wherein the medicament is selected from a vasopressor, an intravenous injection, a TNF-α-antibody, and an antibiotic.
